# EUROPEAN PATENT APPLICATION

(11) **EP 3 626 838 A1**
(43) Date of publication of application: **25.03.2020**
(21) Application number: 18213697.8
(22) Date of filing: 18.12.2018
(51) Int. Cl.: C12R 1/85, C07K 14/39, C12C 11/00

(54) **MALTOTRIOSE METABOLIZING MUTANTS OF SACCHAROMYCES EUBAYANUS**

(30) Priority: 24.09.2018 EP 18196406
(71) Applicant: Heineken Supply Chain B.V., 1017 ZD Amsterdam (NL); Technische Universiteit Delft, 2628 CN Delft (NL)
(72) Inventor: Brouwers, Nick, 2600 AA Delft (NL); de Vries, Arthur Roelof Gorter, 2600 AA Delft (NL); Daran, Jean-Marc Georges, 2600 AA Delft (NL); Kuijpers, Niels Gerard Adriaan, 1017 ZD Amsterdam (NL)
(74) Representative: V.O.

(57) **Abstract**

The invention relates to a mutant of *Saccharomyces eubayanus* that is able to ferment maltotriose, and to the use of this mutant for producing hybrid yeast and the resulting hybrid yeast. The invention relates to methods of producing a fermented beer product by employing said mutant and/or said hybrid yeast.

## Description

### FIELD OF THE INVENTION

The invention relates to the field of microbiology, in particular to the production of novel yeasts and yeast hybrids. The invention further relates to the use of these yeasts and yeast hybrids for producing a fermented beer product, preferably beer.

### 1. BACKGROUND OF THE INVENTION

*Saccharomyces eubayanus* was discovered in Patagonia and identified as the non-S. cerevisiae parental species of lager-type beer brewing *S. pastorianus* hybrids (Libkind et al., 2011. Proc Natl Acad Sci USA 108: 14539-44; Sampaio, 2018. Microbiology 164: 1069-71). While *S. eubayanus* has only been isolated from the wild (Peris et al., 2014. Mol Ecol 23: 2031-45; Bing et al., 2014. Curr Biol 24: R380-R1; Gayevskiy and Goddard, 2016. Environ Microbiol 18: 1137-47), *S. cerevisiae* is strongly associated with human biotechnology, notably for dough leavening, beer brewing and wine fermentation (Dequin, 2001. Appl Environ Microbiol 56: 577-88). Beer brewing is performed on wort, a complex medium containing a fermentable sugar mixture of 60% maltose, 25% maltotriose and 15% glucose (Zastrow et al., 2001. J Ind Microbiol Biotechnol 27: 34-8). While most *S. cerevisiae* strains are able to utilize all three sugars effectively, *S. eubayanus* strains are able to utilize glucose and maltose, but not maltotriose (Hebly et al., 2015. FEMS Yeast Res 15: fov005; Brickwedde et al., 2018. Front Microbiol 9: 1786; Gallone et al., 2016. Cell 166: 1397-410 e16). In *S. cerevisiae*, the ability to utilize maltose and maltotriose is associated with the *MAL* loci: gene clusters which are present on up to five different chromosomes within S. cerevisiae strains (Naumov et al., 1994. Genetics 136: 803-12). *MAL* loci are composed of three genes: *ScMALx1* encoding a maltose proton-symporter, *ScMALx2* encoding an α-glucosidase which hydrolyses sugars into glucose and *ScMALx3* encoding a regulator inducing expression of *ScMALx1* and *ScMALx2* in the presence of maltose (Charron et al., 1989. Genetics122: 307-16). While *ScMALx1* can also transport other disaccharides such as turanose and sucrose (Marques et al., 2017. FEMS Yeast Res 17:fox006; Chang et al., 1989. J Bacteriol 171: 6148-54), they are unable to import the trisaccharide maltotriose (Alves et al., 2008. Appl Environ Microbiol 74: 1494-501). However, the *MAL1* locus located on chromosome VII contains the *ScAGT1* gene encoding a different transporter which only has 57% identity with *ScMALx1* transporter genes (Han et al., 1995. Mol Microbiol 17: 1093-107). *ScAgt1* is a broad substrate specificity proton symporter which enables maltotriose uptake (Alves et al., 2008. Appl Environ Microbiol 74: 1494-501; Stambuk et al., 1999. FEMS Microbiol Lett 170: 105-10). The ability of *S. eubayanus* to utilize maltose is consistent with the presence of four transporters with high homology to *ScMALx1* genes: *SeMALT1, SeMAL2, SeMALT3* and *SeMALT4* (Baker et al., 2015. Mol Biol Evol 32: 2818-31). Deletion of these genes in *S. eubayanus* type strain CBS 12357 indicated that it relies on the expression of *SeMALT2* and *SeMALT4* for maltose transport (Brickwedde et al., 2018. Front Microbiol 9: 1786). *SeMALT1* and *SeMALT3* were poorly expressed in the presence of maltose, supposedly due to incompleteness of the *MAL* loci which harbor them. However, no homolog of *ScAGT1* was found in the genome of type strain CBS 12357, and neither CBS 12357 nor any strain derived from it in which *SeMALT* genes was overexpressed, were able to utilize maltotriose (Brickwedde et al., 2018. Front Microbiol 9: 1786).

In order to reconstruct the putative hybrid ancestor of *S. pastorianus, S. cerevisiae* and *S. eubayanus* strains were mated. Laboratory-made *S. cerevisiae* x *eubayanus* hybrids combined the fermentative capacity and sugar utilization of *S. cerevisiae* with the ability to grow at low temperatures of *S. eubayanus* (Hebly et al., 2015. FEMS Yeast Res 15: fov005; Krogerus et al., 2015. J Ind Microbiol Biotechnol 42: 769-78; Mertens et al., 2015. Appl Environ Microbiol 81: 8202-14). Most likely, maltotriose utilization was due to the *ScAGT1* gene in the *S. cerevisiae* parental genome. Paradoxically, the ability to utilize maltotriose of *S. pastorianus* isolates is not due to *ScAGT1*, as this gene is truncated (Vidgren et al., 2009. Appl Environ Microbiol 75: 2333-45). Instead, maltotriose utilization was associated with two genes specific to *S. pastorianus: SpMTY1* and *SeAGT1.* The gene *SpMTY1* was found in various *S. pastorianus* strains, shares 90% sequence identity with *ScMALx1* genes and enabled maltotriose transport, even with higher affinity than for maltose (Salema-Oom et a., 2005. Appl Environ Microbiol 71: 5044-9; Dietvorst et al., 2005. Yeast 22: 775-88). Interestingly, *SpMTY1* showed sequence similarity with *SeMALT* genes (Cousseau et al., 2013. Lett Appl Microbiol 56: 21-9; Nguyen et al., 2011. PLoS One 6: e25821). *SeAGT1* shares 85% sequence identity with *ScAGT1*, but it was found on *S. eubayanus* chromosome VIII-XV (Nakao et al., 2009. DNA Res 16: 115-29). In accordance with its high sequence similarity, *SeAgt1* displays similar transport properties as *ScAgt1* and also enabled high affinity maltotriose import (Vidgren and Londesborough, 2012. J Inst Brew 118: 148-51). Despite their presence in the *S*. *pastorianus* genome, the maltotriose transporters *SpMty1* and *SeAgt1* were not found during genome sequencing of *S. eubayanus* type strain CBS 12357. While these maltotriose transporters may be present in strains more closely related to the *S*. *eubayanus* ancestor of *S. pastorianus* than CBS 12357 (Bing et al., 2014. Curr Biol 24: R380-1), they may also have evolved during the domestication of *S. pastorianus* in the lager brewing environment.

Regardless of the origin of the *S. eubayanus* maltotriose transporters in *S. pastorianus*, efficient maltotriose utilization is critical for lager brewing. Currently, extensive research is performed for the formation of novel *S. cerevisiae* x *eubayanus* hybrids for industrial lager brewing (Krogerus et al., 2017. Appl Environ Microbiol 101: 65-78) and *S. eubayanus* itself is used for brewing as well (Brickwedde et al., 2018. Front Microbiol 9: 1786). In this context, the inability to utilize maltotriose is not beneficial for their industrial performance, as residual maltotriose influences the beer's flavor profile and sweetness, and the concomitantly lower ethanol yields might limit process profitability (Zheng et al., 1994. J Am Soc Brew Chem 52: 41-7). However, while a maltotriose-utilizing *S. eubayanus* strain would be valuable to the brewing industry, it should not be constructed by targeted genome editing, due to poor customer acceptance of genetically modified organisms (Varzakas et al., 2007. Crit Rev Food Sci Nutr 47: 335-61). Laboratory evolution is a commonly-used non-GMO method to obtain desired properties by prolonged growth and selection under conditions favoring cells which develop the desired phenotype (Mans et al., 2018. Curr Opin Biotechnol 50: 47-56). In *Saccharomyces* yeasts, selectable properties include complex and diverse phenotypes such as high temperature tolerance, efficient nutrient utilization and inhibitor tolerance (Yona et al., 2012. Proc Natl Acad Sci USA 109: 21010-5; Gresham et al., 2008. PLoS Genet 4: e1000303; Gonzalez-Ramos et al., 2016. Biotechnol Biofuels 9: 173; Caspeta et al., 2014. Science 346: 75-8). Laboratory evolution was successfully applied to improve sugar utilization for arabinose, galactose, glucose and xylose (Gresham et al., 2008. PLoS Genet 4: e1000303, Papapetridis et al., 2018. FEMS Yeast Res 18: foy056; Verhoeven et al., 2018. FEMS Yeast Res 18: doi: 10.1093/femsyr/foy062; Hong et al., 2011. Proc Natl Acad Sci USA 108: 12179-84). In *S. pastorianus,* maltotriose uptake was successfully improved by performing chemostat cultivations on medium enriched with maltotriose (Brickwedde et al., 2017. Front Microbiol 8: 1690).

There is thus a need for a maltotriose-utilizing *S. eubayanus* strain that is not constructed by targeted genome editing.

### 2. BRIEF DESCRIPTION OF THE INVENTION

In this study, *S. eubayanus* type strain CBS 12357 was submitted to UV-mutagenesis and laboratory evolution under conditions selecting for the ability to utilize maltotriose. As CBS 12357 is completely unable to grow on maltotriose, UV-mutagenized mutants were first introduced into aerobic shake flasks with synthetic medium with maltotriose as sole carbon source. When growth was observed, sequential aerobic batches were performed under the same conditions until growth was fast and consistent. However, the resulting mutants did not consume any maltotriose when grown on industrial brewing wort. Therefore, the resulting mutants were further evolved during an anaerobic chemostat on brewing wort enriched with maltotriose. The resulting mutants were characterized in industrial wort. The genomes of maltotriose-utilizing mutants were sequenced using short- and long-read sequencing and mutations including SNPs, INDELs and chromosome recombinations were identified. Introduction of putative causal mutations successfully restored maltotriose utilization in the unevolved CBS 12357 strain. As *S. eubayanus* is used in industrial lager brewing, the industrial performance of the non-GMO evolved mutant was evaluated under industrial conditions.

The invention therefore provides a mutant *Saccharomyces* yeast that is able to ferment maltotriose. Said mutant *Saccharomyces* yeast preferably is a mutant *S*. *cerevisiae, S. uvarum, S. bayanus* or *S. eubayanus* yeast. Said mutant *Saccharomyces* yeast preferably comprises a chimeric transporter gene in which part of a first coding gene sequence is translocated adjacent to part of a second coding gene sequence such that the produced chimeric protein harbors part of said first gene product and part of said second gene product. Said mutant *Saccharomyces* yeast preferably comprises sequence elements from *SeMALT1*, *SeMALT2*, *SeMALT3* and/or *SeMALT4,* preferably *SeMALT4*/*SeMALT1*/*SeMALT2* or *SeMALT3.*

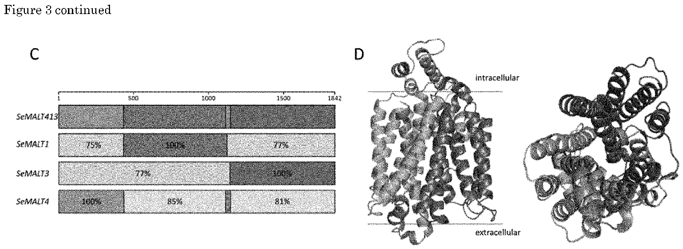

In one embodiment, said *Saccharomyces* yeast is a mutant *S. eubayanus* yeast, having a chimeric maltose transporter gene comprising nucleotides 1-434 of *SeMALT4,* nucleotides 430-1122 of *SeMALT1,* nucleotides 1113-1145 of *SeMALT2* or *SeMALT4,* and nucleotides 1141-1842 of *SeMALT3,* as depicted in Figure 3C. Said mutant *S. eubayanus* yeast preferably has a reduced decarboxylation activity of phenolic acids, preferably is not producing 4-vinyl guaiacol.

The invention further provides a method for producing a hybrid yeast, comprising a) providing the mutant *S. eubayanus* yeast of any one of claims 1-5 as a first parent, and a second yeast as a second parent, which said second parent differs from the first parent, b) hybridizing cells from the first parent with cells from the second parent and c) identifying a resulting hybrid organism. Said second parent is a yeast of the *Saccharomyces* sensu stricto complex.

In a preferred method of the invention, the cells from the first and/or second parent are labeled with a fluorescent dye, prior to hybridizing the cells. In a further preferred method, the hybridization is performed at a temperature that is at least 5 °C below the optimal growth temperature of the first and/or the second parent.

The invention further provides a hybrid yeast, produced by a method for producing a hybrid yeast according to the invention.

The invention further provides a method of producing a fermented beer product, comprising the steps of adding a fermentative yeast according to the invention into a wort, and at least partially fermenting said wort to produce a fermented beer product.

Said fermentative yeast preferably comprises a mutation resulting in inactivation of at least one of the genes *PAD1* and *FDC1,* and/or inactivation of a gene encoding a protein involved in uptake of a phenolic acid, preferably ferulic acid, or involved in export of a decarboxylated phenolic compound, preferably 4-vinyl guaiacol. Said produced fermented beer product preferably is beer, preferably a lager beer. In a preferred method of producing a fermented beer product, the alcohol content of the fermented beer product is reduced after fermentation, preferably by rectification evaporation.

The invention further provides a fermented beer product that is produced by the methods of the invention.

The invention further provides an use of a mutant *Saccharomyces* yeast according to the invention for producing a hybrid yeast.

The invention further provides an use of a mutant *Saccharomyces* yeast or of a hybrid yeast according to the invention for producing a fermented beer product, preferably a beer, more preferably lager beer.

### 3. FIGURE LEGENDS

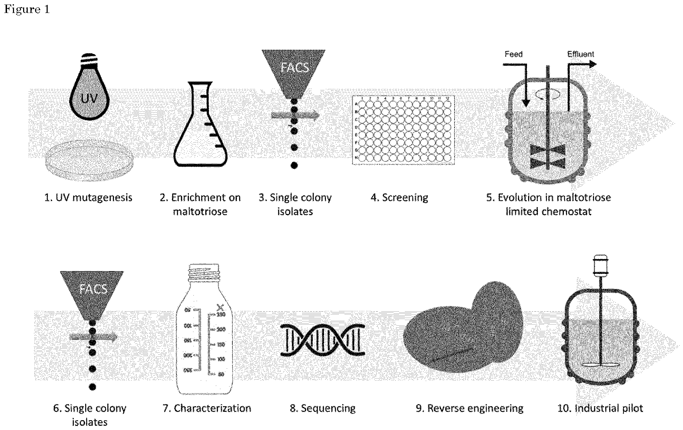

Figure 1. Schematic overview of process to develop industrial relevant maltotriose utilization in *S. eubayanus* CBS 12357. Sporulated *S. eubayanus* cells were irradiated with UV to generate novel phenotypes. The mutant pool was enriched for maltotriose-consuming cells by growth on SM with maltotriose as sole carbon source. From the enriched culture, single colony isolates were made using FACS which were subsequently screened for high OD₆₆₀ in microtiter plates. To enhance obtained isolates for maltotriose consumption under industrial relevant conditions, 7 mutants were pooled and evolved in a carbon limited anaerobic chemostat on modified industrial wort enriched with additional maltotriose. After evolution, single colony isolates were generated using FACS and characterized under industrial relevant conditions. Successful evolved mutants were whole genome sequenced and resulting translocations were reverse engineered by overexpression of *SeMALT413* in CBS 12357. Finally, to demonstrate applicability and industrial relevance, the evolved mutant was tested on an industrial pilot scale.

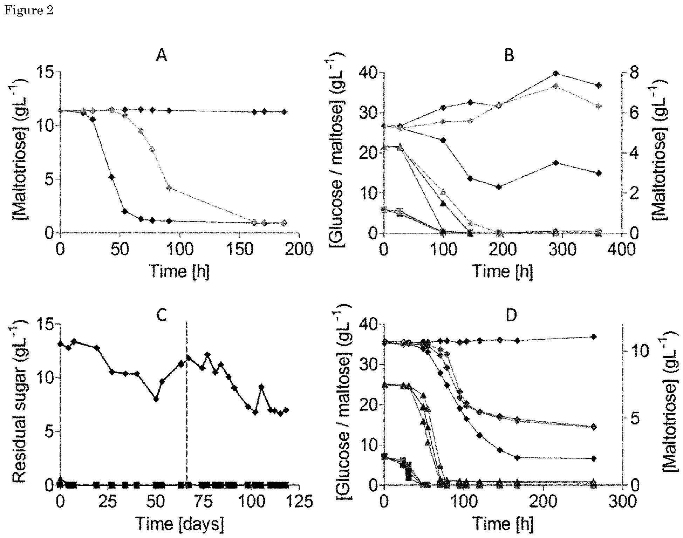

Figure 2. Mutagenesis and evolution to obtain maltotriose consuming *S*. *eubayanus.* (A) Characterization of *S. pastorianus* CBS 1483 (▲), *S. eubayanus* CBS 12357 (■) and IMS0637 (●) on SMMt at 20 °C. The data for IMS0637 is representative for the other mutants IMS0638-IMS0643. The average concentration of maltotriose (◆) and average deviation were determined from two replicates. (B) Characterization of *S. pastorianus* CBS 1483 (black), *S. eubayanus* CBS 12357 (white) and IMS0637 (grey) on wort at 20 °C. The concentrations of (■) glucose, (▲) maltose and (◆) maltotriose were measured from single biological measurements. (C) Residual maltotriose concentration in the outflow during laboratory evolution of strains IMS0637-IMS0643 in an anaerobic chemostat at 20 °C on maltotriose enriched wort. The concentrations of (■) glucose, (▲) maltose and (◆) maltotriose were measured by HPLC. The chemostat was restarted after a technical failure (dotted line). (D) Characterization of *S. pastorianus* CBS 1483 (black), *S. eubayanus* CBS 12357 (white), IMS0750 (dark grey) and IMS0752 (light grey) on wort at 12 °C in 250 mL micro-aerobic bottles. The average concentration and standard deviation of (■) glucose, (▲) maltose and (◆) maltotriose were determined from three biological replicates.

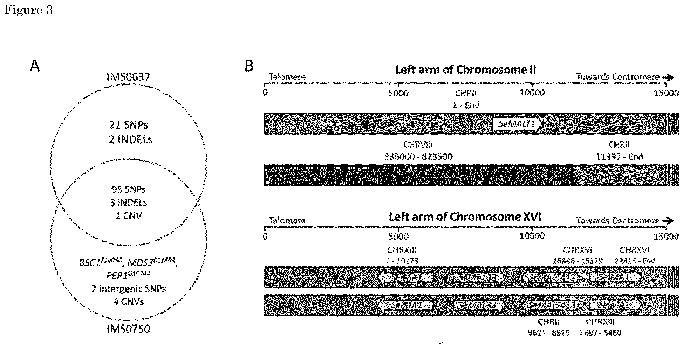

Figure 3: Identification of mutations in the mutagenized strain IMS0637 and the evolved strain IMS0750. (A) Venn diagram of the mutations found in UV-mutagenized IMS0637 and evolved IMS0750 relative to wildtype CBS 12357. Single nucleotide polymorphisms (SNPs), small insertions and deletions (INDELs) and copy number variation (CNV) are indicated as detected by Pilon. (B) Recombined chromosome structures in IMS0637 and IMS0750 as detected by whole genome sequencing using MinION technology and de novo genome assembly. The first 15'000 nucleotides of the left arm of CHRII and CHRXVI are represented schematically. The origin of the sequence is indicated in black for CHRII, marked for CHRVIII, light grey for CHRXIII and dark grey for CHRXVI. In addition, *SeMALT* transporter genes present on the sequence are indicated by arrows. While the recombination of CHRII and CHRVIII was present in IMS0637 and IMS0750, the recombination of both copies of CHRXVI was found only in IMS0750 but not in IMS0637. The recombination on CHRXVI created the chimeric SeMALT413 transporter. (C) Overview of the sequence similarity of the 1842 nucleotides of *SeMALT413* relative to *SeMALT1*, *SeMALT3* and *SeMALT4.* The open reading frames of the genes were aligned and regions with 100% sequence identity were identified. For regions in which the sequence identity was lower than 100%, the actual sequence identity is indicated for each *SeMALT* gene. The origin of the sequence is indicated in black for CHRII, marked for CHRVIII, light grey for CHRXIII and dark grey for CHRXVI. (D) Prediction of the protein structure of SeMalt413 with on the left side a transmembrane view and on the right a transport channel view. Domains originated from *S. eubayanus SeMalt* transporters are indicated by the colors dark grey (*Se*Malt4 chromosome XVI), black (*Se*Malt1 chromosome II) and light grey (*Se*Malt3 chromosome XIII).

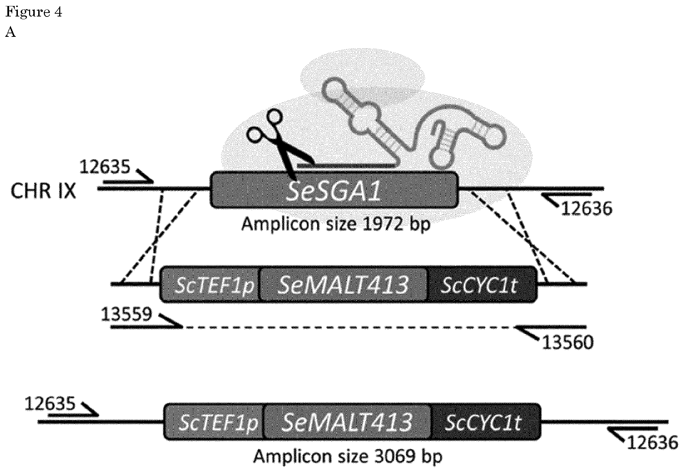

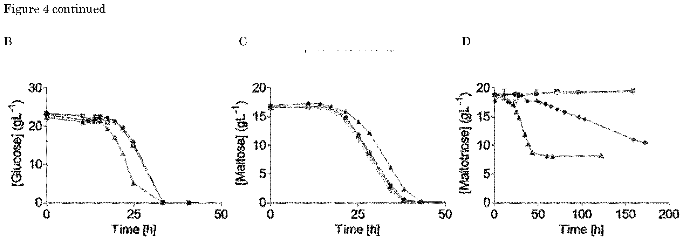

Figure 4: Reverse engineering of *SeMALT413* in CBS 12357 and characterization of transporter functionality in SM. (A) Representation of the CRISPR-Cas9 gRNA complex (after self-cleavage of the 5' hammerhead ribozyme and a 3' hepatitis-δ virus ribozyme from the expressed gRNA) bound to the *SeSGA1* locus in CBS 12357. Repair fragment with transporter cassette *ScTEF1*p-*SeMALT413-ScCYC1t* was amplified from pUD814(*Se*MALT413) with primers 13559/13560 and contains overhangs with the *SeSGAl* locus for recombination. *SeSGA1* was replaced by the *ScTEF1*p-*SeMALT413-ScCYC1*t cassette. Correct transformants were checked using primers 12635/12636 upstream and downstream of the *SeSGA1* locus. Strains were validated using Sanger sequencing. Characterization of (■)CBS 12357, **(▲)** IMS0750, (▼) IMX1941, (◆) IMX1942 on SM (B) glucose, (C) maltose and (D) maltotriose. Strains were cultivated at 20 °C and culture supernatant was measured by HPLC. Data represent average and standard deviation of three biological replicates.

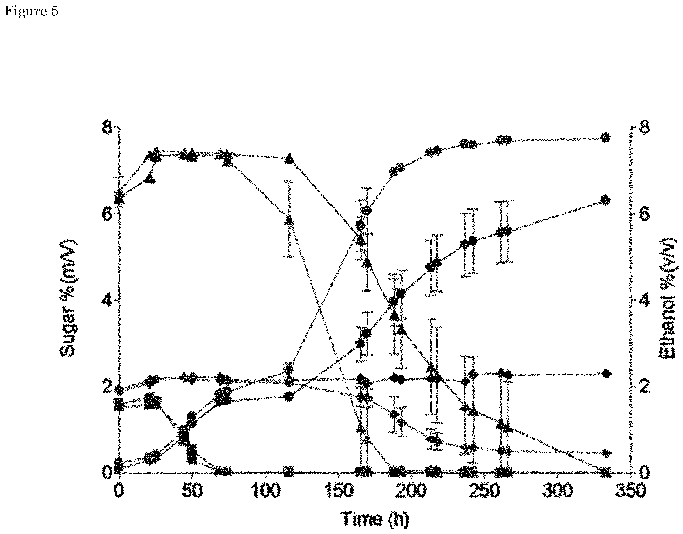

Figure 5: Extracellular metabolites profiles of *S. eubayanus* strains CBS 12357 (black) and IMS0750 (white) in high- gravity wort (17 °P) at 7-L pilot scale. (A) Sugars consumption and ethanol production. The sugars time course data expressed in % (m/v) are represented as follow: glucose (■), maltose (▲), maltotriose (◆). The ethanol production profiles expressed in % (v/v) are represented as (●).

### 4. DETAILED DESCRIPTION OF THE INVENTION

### 4.1 Definitions

The term "fermented beer product", as is used herein, refers to a beer product that is produced by fermentation of, for example, crops and products thereof such as grains, rice, grapes and other fruits, nuts and/or exudations from, e.g. agave, yucca and cactus.

The term "alcohol-reduced fermented beer product", as is used herein, refers to a fermented beer product having a reduced level of ethanol, when compared to a corresponding normal fermented beer product, For example, an alcohol-reduced beer preferably comprises less than 5 vol %, such as 0.5-1.2% vol % of ethanol as an alcohol.

The term "alcohol-free fermented beer product", as is used herein, refers to a fermented beer product in which no ethanol is present, or in which less than 0.03 vol % is present. It is noted that the maximal percentage for an alcohol-free beer may differ between countries. For example, alcohol-free beer, also termed "non-alcoholic beer", may contain less than 0.5 vol % in the USA and some European countries, but not more than 0.05 vol % in the UK. However, as used herein, the term "alcohol-free fermented beer product" refers to a fermented beer product in which no ethanol is present, or in which less than 0.03 vol % is present.

The term "maltotriose", as is used herein, refers to a trisaccharide consisting of three glucose molecules linked through α-1,4 glycosidic bonds.

The term "decarboxylation activity of phenolic acids", as is used herein, refers to the amount of phenolic acids that is converted to its decarboxylated form, preferably the amount of phenolic acids that is enzymatically converted to its decarboxylated form. Enzymatic conversion is preferably catalysed by at least one or both of the two proteins encoded by the genes encoding phenylacrylic acid decarboxylase (*PAD1*) and/or ferulic acid decarboxylase (*FDC1*)*.* It has been shown that inactivation of one of these two genes is sufficient to interfere with decarboxylation of phenolic acids. Decarboxylation activity of phenolic acids, i.e. the amount of phenolic acids that is converted to its decarboxylated form can be determined by any method known in the art. For example, ferulic acid and 4-VG display a strong difference of their light absorption spectra between 200 and 400 nm. Ferulic acid shows high absorption values above 300 nm, while conversion into 4-VG results in a decrease of absorption values above 300 nm. This difference may be used to estimate the conversion capacity of ferulic acid into 4-VG, as an estimate for the decarboxylation activity of phenolic acids. For instance, the supernatant of e.g. microtiter plate cultures grown in synthetic wort in the presence of ferulic acid can be collected by centrifugation, e.g. for 5 minutes at 2500xg at 4°C, transferred to a microtiter plate and an absorption spectrum from 250 nm to 400 nm of the 96 well microtiter plate can be determined. As another example, decarboxylation activity can be determined by incubating a yeast cell, or a culture of yeast cells, in the presence of substrate, i.e. a phenolic acid such as ferulic acid or cinnamic acid, and determining the conversion of the phenolic acid to its decarboxylated form by mass spectrometry or high performance liquid chromatography (HPLC).

The term "reduced decarboxylation activity of phenolic acids", as is used herein, refers to the percentage of decarboxylation activity of a yeast, which is reduced when compared to a control, preferably an unmodified control. The conversion of phenolic acids can for instance be determined during a predetermined period of time and compared to the conversion of phenolic acids in a control yeast cell or culture of yeast cells during the same period of time. As another example, decarboxylation activity can be determined in a more indirect way by determining the ratio of proliferation of yeast cells cultured in the presence of cinnamic acid and the proliferation of yeast cells cultures in the absence of cinnamic acid. Since cinnamic acid is more toxic to yeast cells than its decarboxylated form styrene, a reduced proliferation of yeast cells in the presence of cinnamic acid of a yeast cell or culture of yeast cells as compared to a reference, means that the decarboxylation activity is reduced. The percentage reduction can for instance be determined by determining the ratio of proliferation of yeast cells cultured in the presence of cinnamic acid. Alternatively, proliferation of yeast cells in the presence or absence of cinnamic acid can be determined and the ratio of proliferation of yeast cells cultured in the presence of cinnamic acid and the proliferation of yeast cells cultures in the absence of cinnamic acid can be determined as a measure of decarboxylation activity. As a reference, a normal yeast strain that is routinely used in fermentation processes, for example a the Heineken- A yeast and/or the Heineken D- yeast for beer fermentation, may be used as a reference for determining a reduced decarboxylation activity of phenolic acids. Said reduction preferably is at least 50%, more preferably at least 60%, more preferably at least 70%, more preferably at least 80%, more preferably at least 90%, more preferably at least 99%, when compared to a normal yeast strain that is routinely used in the indicated fermentation process. This means that a yeast having a reduced decarboxylation activity of phenolic acids has a decarboxylation activity that is at most 40% of the decarboxylation activity of a reference, more preferably at most 30%, more preferably at most 25%, more preferably at most 20%, more preferably at most 15%, more preferably at most 10%, more preferably at most 5%, most preferably at most 1% of the decarboxylation activity of said reference.

The term "mutation", as is used herein, refers to an alteration in the genomic DNA of a yeast, including, but is not limited to, a point mutation, an insertion or deletion of one or more nucleotides, a substitution of one or more nucleotides, a frameshift mutation, and single stranded or doubled stranded DNA break, such as a chromosome break or translocation, and any combination thereof.

The term "translocation", as is used herein, refers a chromosomal segment is moved from one position to another, either within the same chromosome or to another chromosome. A translocation may be reciprocal, meaning that fragments are mutually exchanged between two chromosomal location, such as between two chromosome.

The term "gene", as is used herein, refers to any and all cis-acting genomic sequences that ensure that a product encoded by the gene is expressed, including enhancer and promotor sequences, exonic and intronic sequences. Said product is may be an RNA molecule, such as a mRNA molecule or an siRNA molecule, and/or a protein.

The term "a gene involved in transcriptional control" of another gene, as is used herein, refers a gene encoding a transcriptional regulator or factor that regulates expression of that other gene.

The term "inactivated gene", as is used herein, indicates a gene that is not able to perform its normal function. E.g. for a gene encoding a protein "inactivation" means that the gene does not translate into a protein, encodes an inactive protein or encodes a protein with reduced activity. Said inactivation, for example, may be due to an alteration in a promoter sequence such that the promoter is not capable of initiating transcription of the gene, to an alteration of a splicing site of an intron, which alteration interferes with correct splicing of the transcribed pre-mRNA, or an alteration in the coding region of the gene, rendering the encoded protein less active or even inactive. Said inactivation preferably is at least 50%, more preferably at least 60%, more preferably at least 70%, more preferably at least 80%, more preferably at least 90%, more preferably at least 99%, when compared to not inactivated gene.

The term "promoter", as is used herein, refers to a genomic sequence that is considered as a regulatory region of a gene that is required for initiating transcription thereof. It is typically located in the 5' part of the gene, typically but not exclusively in front of the transcription start site.

The term "hybrid" or "hybrid yeast", as is used herein, refers to a yeast that is the result of combining genomes of two yeast of different varieties or species. A hybrid preferably is the result of sexual crossing, meaning that the hybrid yeast is the result of fusion of two cells of different sex, such as two cells of different mating types, preferably of two gametes.

The term "interspecies hybrid ", as is used herein, refers to a yeast that is the result of combining genomes of two organisms of different species or genera.

The terms "first parent" and "second parent", as are used herein, refer to two yeasts of different varieties or species. Said two yeasts are hybridization-compatible.

The term "hybridization-compatible", as is used herein, refers to two yeasts that can be crossed, preferably sexually crossed. The term "mating compatible" may be used, which equals the term "hybridization-compatible".

The terms "dye A" and "dye B" refer to different fluorescent dyes that can be used to stain yeast cells.

The term "optimal growth temperature", as is used herein, refers to the temperature at which the yeast cells from a first parent organism and from a second parent organism growth optimally, meaning that cells complete a full cell cycle fastest. Most yeast have an optimal growth temperature between 10 and 40 °C, preferably between 15 and 30 °C, such as between 18 °C and 25 °C, more specifically between 20 °C and 22 °C.

The term "auxotrophic marker", as is used herein, refers to marker genes that encode key enzymes in metabolic pathways towards essential metabolites, especially monomers, used in biosynthesis. An example is the *URA3* gene, which encodes orotidine-5'-phosphate decarboxylase, an essential enzyme in pyrimidine biosynthesis in *Saccharomyces cerevisiae.* Similarly, *HIS3, LEU2, TRP1,* and *MET15* marker genes encode essential enzymes for de novo synthesis of the amino acids histidine, leucine, tryptophan, and methionine, respectively. The presence of an auxotrophic marker allows growth of cells in the absence of the corresponding essential metabolite.

The term "diploid", as is used herein, refers to a cell or an organism comprising of two sets of chromosomes. One set of chromosomes is obtained from one parent, while a second set of chromosomes normally is obtained from a second parent. The term "diploid" is used to separate cells and organisms having two sets of chromosomes, from cells and organisms having one set of chromosomes, termed haploid, and from cells and organisms having multiple sets of chromosomes, termed polyploid. Polyploid cells and organisms include triploid, tetraploid, pentaploid, hexaploid and octaploid cells and organisms.

The term "aneuploid", as is used herein, refers to a cell or an organism in which not all chromosomes are present in the same number of copies. Hence, the chromosome complement can not be indicated as a defined number of complete chromosome sets, such as n, 2n, 3n, or 4n, as is known to a person skilled in the art. The term aneuploidy refers to the presence of an abnormal number of chromosomes in a cell or organism, in contrast to an euploid cell. An aneuploid cell may miss or have an extra part of a chromosome, or may miss one or more chromosome or have one or more chromosomes extra.

The term "germination", as is used herein, refers to the process by which a seed or a gamete recovers the ability to grow vegetatively, resulting in multicellular structures or in cell replication by mitotic growth. The most common example of germination is the sprouting of a seedling from a seed. In addition, the growth of a sporeling from a spore, such as the spores of hyphae from fungal spores, is also termed germination. In addition, the process in which a fungal spore sheds its spore wall and recovers normal metabolic activity, such as occurs in yeasts is also termed germination. Germination often depends on conditions such a temperature, humidity, oxygen supply and sometimes light or darkness.

The terms "yeast" and fermentative yeast", as are used herein, refer to eukaryotic, unicellular microorganisms that are classified as members of the kingdom fungus. A preferred yeast is a yeast of the *Saccharomyces* sensu stricto complex, including any hybrid thereof. The *Saccharomyces* sensu stricto complex currently encompasses nine different species: *Saccharomyces cerevisiae, S. paradoxus, S. cariocanus, S. uvarum, S. mikatae, S. kudriavzevii, S. arboricola, S. eubayanus* and the recently discovered *S. Jurei* [Hittinger, 2013. Trends Genet 29: 309-317; Naseeb et al., 2017. Int J Syst Evol Microbiol 67: 2046-2052].

The term "fermentative yeast", as is used herein, refers to a yeast of the *Saccharomyces* sensu stricto complex, preferably a *Saccharomyces cerevisiae or S.* *eubayanus* yeast, and/or a hybrid thereof such as *S. pastorianus,* also termed *S*. *carlsbergensis.*

### 4.2 Method of selecting a maltotriose utilizing mutant of a non maltotriose utilizing

### Saccharomyces

Mutagenesis of *Saccharomyces yeast* can be performed using any method known in the art, including conventional random mutagenesis methods, such as radiation and chemical treatment, and recombinant DNA technologies, such as site-directed mutagenesis or targeted mutagenesis. Hence, the yeast cell may have been subjected to random mutagenesis, including treatment with UV irradiation, X-ray irradiation, gamma-ray irradiation and a mutagenic agent, or to genetic engineering.

The term "random mutagenesis" refers to mutagenesis techniques whereby the exact site of mutation is not predictable, and can occur anywhere in the chromosome of the yeast cell(s) or spore(s). In general, these methods involve the use of chemical agents or radiation for inducing at least one mutation. Random mutagenesis can further be achieved using error prone PCR wherein PCR is performed under conditions where the copying accuracy of the DNA polymerase is low, resulting in a relatively high rate of mutations in the PCR product.

"Genetic engineering" is well known in the art and refers to altering the yeast's genome using biotechnological method, thereby introducing an alteration of the genomic DNA of the yeast, preferably at a predefined site and with a predefined alteration, termed site-directed mutagenesis.

Targeted mutagenesis, also termed site-directed mutagenesis, can be achieved using oligonucleotide-directed mutagenesis to generate site-specific mutations in a genomic DNA sequence of interest. Targeted mutagenesis refers to a mutagenesis method that alters a specific gene in vivo resulting in a change in the genetic structure directed at a specific site, such as by programmable RNA-guided nucleases, such as TALEN, CRISPR-Cas, zinc finger nuclease or meganuclease technology.

Said mutagenesis preferably is performed by subjecting a yeast to treatment with radiation, such as UV irradiation, X-ray irradiation, gamma-ray irradiation, and/or a mutagenic agent, preferably a chemical agent such as NTG (N-methyl-N'-nitro-N- nitrosoguanidine) or EMS (ethylmethanesulfonate). A particularly preferred mutagenesis procedure comprises UV irradiation, e.g. for 10 seconds to 3 minutes, preferably approximately 1-2 minutes. A preferred method includes exposure to UV light (TUV 30 W T8, Philips, Eindhoven, The Netherlands) at a radiation peak of 253,7 nm and for a period of 0.1 to 10 minutes, preferably 0.5-5 minutes, such as about 90 minutes.

Said mutagenesis, preferably random mutagenesis, preferably is performed in two or more rounds. Each round preferably includes a mutagenesis step, preferably a mild mutagenesis step, preferably a UV-mediated mutagenesis step, which results in a moderate survival rate of 20-60%, preferably 40-50%.

In a first round, the mutated yeasts may by inoculated in a synthetic medium containing maltotriose as the sole carbon source, which will enrich for mutants that are able to consume maltotriose.

A second round of mutagenesis preferably includes growth on brewer's wort that is enriched with maltotriose. Under these conditions, mutants with an improved affinity or an higher transport rate for growth on maltotriose would be less nutrient-limited, resulting in a selective advantage when compared to not-mutated yeasts. For this, wort may be diluted 2-10 times, for example six-fold. Said diluted wort may be supplemented with maltotriose, for example 1-20 g L⁻¹ such as 10 g L⁻¹ of maltotriose, to increase the relative concentration of maltotriose. Ergosterol, for example 1-100 mg L⁻¹, TWEEN® 80, for example 100-1000 mg L⁻¹, and ammonium sulfate, for example 1-20 mg L⁻¹, may be supplemented to prevent oxygen and nitrogen limitation.

Said growth on maltotriose-enriched brewer's wort is preferably performed by a continuous culture. Said continuous culture may be operated at a dilution rate of 0.001-0.2 h⁻¹, preferably at 0.01-0.1 h⁻¹ such as 0.03 h⁻¹. At a time point that maltotriose concentration decreases, single cells from the culture are preferably isolated, for example by FACS sorting. The isolated cell may be plated on synthetic medium containing maltotriose as the sole carbon source, and/or on brewer's wort that is enriched with maltotriose as is described herein above to further select *Saccharomyces* mutants, preferably *S. eubayanus* mutants, that can utilize maltotriose.

Further *Saccharomyces* yeast that cannot utilize maltotriose include some *S*. *cerevisiae* strains, *S. uvarum* and *S. bayanus.* The above described methods of selecting a maltotriose utilizing mutant of a non maltotriose utilizing *Saccharomyces* are applicable to *S. uvarum* and *S. bayanus,* in addition to *S. eubayanus.*

### 4.3 Mutant Saccharomyces yeast utilizing maltotriose

*S. eubayanus* was first isolated from Nothofagus trees and stromata of Cyttaria harioti in North-Western Patagonia (Libkind et al., 2011. Proc Natl Acad Sci 108: 14539-44). Strains of *S. eubayanus* have subsequently been also isolated from locations in North America (Peris et al., 2014. Mol Ecol 23: 2031-45), Asia (Bing et al., 2014. Curr Biol 24: R380-1) and Oceania (Gayevskiy and Goddard, 2016. Environ Microbiol 18: 1137-47). Initial physiological characterization of the Patagonian *S*. *eubayanus* strain CBS12357^{T} revealed that it grows faster than *S. cerevisiae* at temperatures below 10 °C (Hebly et al., 2015. FEMS Yeast Res 15: fov005), shows poor flocculation (Krogerus et al., 2015. J Ind Microbiol Biotechnol 42: 769-78) and consumes maltose but not maltotriose (Gibson et al., 2013. Yeast 30: 255-266). Gibson et al., 2017. FEMS Yeast Res 17: fox038; Hebly et al., 2015. FEMS Yeast Res 15: fov005).

Most *S. eubayanus* strains are not capable of transporting maltotriose and/or converting maltotriose into ethanol.

The genome of *S. eubayanus* harbors nine genes annotated as hexose facilitator (*HXT*) transporter orthologs (Baker et al., 2015. Mol Biol Evol 32: 2818-2831; Hebly et al., 2015. FEMS Yeast Res 15: fov005). In addition to these energy-independent hexose facilitators, a fructose/H+ symporter, is present in *S. eubayanus* (Pengelly and Wheals, 2013. FEMS Yeast Res 13: 156-161). The genome of *S. eubayanus* type strain CBS 12357 further harbors four *Se*Malt maltose transporters, termed *SeMALT1*; *SeMALT2, SeMALT3,* and *SeMALT4* which, however, have not yet been functionally analysed (Baker et al., 2015. Mol Biol Evol 32: 2818-2831). None of them appear to transport maltotriose since *S. eubayanus* CBS12357 is unable to grow on this trisaccharide (Hebly et al., 2015. FEMS Yeast Res 15: fov005). *S. eubayanus* does not seem to encode any transporter with high similarity to the *S. cerevisiae* maltotriose transporter *Sc*Agt1, or to the *S. pastorianus* maltotriose transporters *Sp*Mty1 and *Se*Agt1 (Baker et al., 2015. Mol Biol Evol 32: 2818-2831; Hebly et al., 2015. FEMS Yeast Res 15: fov005), although a gene having 81% of homology to the *AGT1* permease from *S. cerevisiae* has been reported (Cousseau et al., 2012. Letters in Applied Microbiology 56: 21-29).

Said mutant yeast preferably is of the *Saccharomyces* sensu stricto complex that comprises a gene encoding an activated transporter as described, and any chimeric genes paralogous and homologous to these transporters in *Saccharomyces* genomes.

A preferred mutant *S. eubayanus* yeast according to the invention is obtained after random mutagenesis, preferably after UV mutagenesis.

Mutagenesis of *S. eubayanus* may result in activation of one or more transporter genes that, after activation, are able to transport maltotriose, and/or activation of one or more intracellular α-glucosidases.

Said activated transporter genes may include known maltose transporter genes in *S. cerevisiae,* or their homologues in *S. eubayanus,* such as *MPH2, MPH3* and *MALx1* genes, including *MAL21, MAL31, MAL41, MAL61, AGT1* (also referred to as *MAL11*)*.* Said activation may further, or in addition, include activation of a *MTY1* homologue (also referred to as *MTT1*) from *S. pastorianus* in *S. eubayanus,* and/or *AGT1* and *MALT* genes of *S. eubayanus,* including *SeMALT1, SeMALT2, SeMALT3, SeMALT4.*

Mutagenesis of *S. eubayanus* may result in an alteration in one or more transporter genes, including the nine *HXT* genes, the fructose/H+ symporter and/or the four *Se*Malt maltose transporters, and/or result in one or more alterations resulting in, for example, activation of a yet unknown maltotriose transporter, activation of an upstream transcriptional activator, and/or inactivation of a transcriptional repressor of one or more of the above indicated known or unknown transporter genes.

Similarly, mutagenesis of *S. eubayanus* may result in an alteration of one or more genes of which the encoded products are involved in the breakdown of maltotriose into glucose. Hydrolysis of maltotriose into glucose is facilitated by intracellular α-glucosidases, also termed maltases, which hydrolyze terminal 1, 4-linked α-D-glucose residues, thereby releasing α-D-glucose. Three different α-glucosidases have been isolated from brewer's yeast, of which two proteins are capable of hydrolyzing maltotriose (Matsusaka et al., 1977. Agric Biol Chem 41: 1917-1923). A priori, alteration of genes that result in activation of one or more intracellular α-glucosidases may result in a mutant *S. eubayanus* yeast that is able to ferment maltotriose. Said mutation resulting in activation of one or more intracellular α-glucosidases may be separate to, or in addition to, one or more mutations that result in activation of at least one transporter of maltotriose in a *S. eubayanus* yeast.

In addition, alteration of a cell surface glucose sensor Rgt2 and/or Snf3, and or of the downstream nuclear transcription factor Rgt1, can be employed to repress genes encoding glucose transporters (Roy et al., 2016. Mol Biol Cell 27: 862-871). A person skilled in the art will understand that alteration, preferably by random mutagenesis, of one or more genes encoding key enzymes in uptake, fermentation and/or aerobic degradation of maltotriose in *S. eubayanus,* may result in a fermentative *S*. *eubayanus* yeast that is capable of converting maltotriose into ethanol, preferably of completely converting maltotriose that is present, for example in wort, into ethanol.

The invention therefore provides a mutant *Saccharomyces eubayanus* yeast that is able to ferment maltotriose.

Said mutant *S. eubayanus* yeast preferably comprises a chimeric gene in which part of a first coding gene sequence is translocated adjacent to part of a second coding gene sequence such that the produced protein harbors part of said first gene product and part of said second gene product. For example, said chimeric gene may encode a first part of *Se*Malt4 and a second part of *Se*Malt1. Said chimeric protein preferably comprises a combination of *Se*Malt 1 and *Se*Malt 4 amino acid sequences, denoted as *Se*Malt 1/ *Se*Malt 4 protein in which the N-terminal part is provided by *Se*Malt1 and a C-terminal part is provided by *Se*Malt4, or a *Se*Malt1/*Se*Malt2 protein, a *Se*Malt1/*Se*Malt3 protein, a *Se*Malt2/*Se*Malt1 protein, a *Se*Malt2/*Se*Malt3 protein, a *Se*Malt2/*Se*Malt4 protein, a *Se*Malt3/*Se*Malt1 protein, a *Se*Malt3/*Se*Malt2 protein, a *Se*Malt3/SeMalt4 protein, a *Se*Malt4/*Se*Malt1 protein, a *Se*Malt4/*Se*Malt2 protein, or a *Se*Malt4/*Se*Malt3 protein.

Said chimeric protein may comprise a combination of three SeMalt amino acid sequences including, for example, *SeMalt*1/*SeMalt*2/*SeMalt*3*, SeMalt*1/*SeMalt*3/*SeMalt*3, *SeMalt*1/*SeMalt*2/*SeMalt*4*, SeMalt*1/*SeMalt*3/*SeMalt*4, *SeMalt*2/*SeMalt*1/*SeMalt*3, *SeMalt*2/*SeMalt*1/*SeMalt*4*, SeMalt*2/*SeMalt*3/*SeMalt*1, *SeMalt*2/*SeMalt*3/*SeMalt*4*, SeMalt*2/*SeMalt*4/*SeMalt*1*, SeMalt*2/*SeMalt*4/*SeMalt*3*, SeMalt*3/*SeMalt*1/*SeMalt*2*, SeMalt*3/*SeMalt*1/*SeMalt*4*, SeMalt*3/*SeMalt*2/*SeMalt*1*,* *SeMalt*3/*SeMalt*2/*SeMalt*4*, SeMalt*3/*SeMalt*1/*SeMalt*2, *SeMalt*3/*SeMalt*1/*SeMalt*4*, SeMalt*4/*SeMalt*1/*SeMalt*2, *SeMalt*4/*SeMalt*1/*SeMalt*3*, SeMalt*4/*SeMalt*2/*SeMalt*1*, SeMalt*4/*SeMalt*2/*SeMalt*3, *SeMalt*4/*SeMalt*3/*SeMalt*1, or *SeMalt*4/*SeMalt*3/*SeMalt*2.

Because *SeMALT2* and *SeMALT4* are known to be expressed in *S. eubayanus* yeast, the N-terminal part of the chimeric protein preferably is from either *SeMALT2* or from *SeMALT4.* Accordingly, a further preferred chimeric protein comprises, for example, *SeMalt*2/*SeMalt*1/*SeMalt*3*, SeMalt*2/*SeMalt*1/*SeMalt*4*, SeMalt2*/*SeMalt3*/*SeMalt*1, *SeMalt*2/*SeMalt*3/*SeMalt*4, *SeMalt*2/*SeMalt*4/*SeMalt*1, *SeMalt*2/*SeMalt*4/*SeMalt*3, *SeMalt*4/*SeMalt*1/*SeMalt*2, *SeMalt*4/*SeMalt*1/*SeMalt*3, *SeMalt*4/*SeMalt*2/*SeMalt*3, *SeMalt*4/*SeMalt*2/*SeMalt*3, *SeMalt*4/*SeMalt*3/*SeMalt*1/*SeMalt*3, *SeMalt*4/*SeMalt*3/*SeMalt*2/*SeMalt*3, *SeMalt*2/*SeMalt*1/*SeMalt*4/*SeMalt*3, *SeMalt*2/*SeMalt*3/*SeMalt*1/*SeMalt*3, *SeMalt*2/*SeMalt*3/*SeMalt*4/*SeMalt*3, *SeMalt2*/*SeMalt4*/*SeMalt*1/*SeMalt*3, *SeMalt*4/*SeMalt*3/*SeMalt*1/*SeMalt*3, *SeMalt*4/*SeMalt*3/*SeMalt*1/*SeMalt*3, *SeMalt*4/*SeMalt*3/*SeMalt*1/*SeMalt*3, *SeMalt4*/*SeMalt*3/*SeMalt*2/*SeMalt*3, *SeMalt*4/*SeMalt*3/*SeMalt*1/*SeMalt*3, *SeMalt4*/*SeMalt*3/*SeMalt*2/*SeMalt*3, *SeMalt*2/*SeMalt*1/*SeMalt*3/*SeMalt*1, *SeMalt*2/*SeMalt*1/*SeMalt*4/*SeMalt*1*, SeMalt*2/*SeMalt*3/*SeMalt*4/*SeMalt*1, *SeMalt*2/*SeMalt*4/*SeMalt*3/*SeMalt*1*, SeMalt*4/*SeMalt*1/*SeMalt*2/*SeMalt*1, *SeMalt*4/*SeMalt*1/*SeMalt*3/*SeMalt*1, *SeMalt*4/*SeMalt*2/*SeMalt*3/*SeMalt*1, *SeMalt*4/*SeMalt*3/*SeMalt*1/*SeMalt*1, *SeMalt*4/*SeMalt*3/*SeMalt*2/*SeMalt*1, *SeMalt*2/*SeMalt*1/*SeMalt*3/*SeMalt*2, *SeMalt*2/*SeMalt*1/*SeMalt*4/*SeMalt*2, *SeMalt*2/*SeMalt*3/*SeMalt*1/*SeMalt*2, *SeMalt*2/*SeMalt*3/*SeMalt*4/*SeMalt*2, *SeMalt*2/*SeMalt*4/*SeMalt*1/*SeMalt*2, *SeMalt*2/*SeMalt*4/*SeMalt*3/*SeMalt*2, *SeMalt*4/*SeMalt*1/*SeMalt*2/*SeMalt*4, *SeMalt*4/*SeMalt*1/*SeMalt*3/*SeMalt*4, *SeMalt*4/*SeMalt*2/*SeMalt*1/*SeMalt*4, *SeMalt*4/*SeMalt*2/*SeMalt*3/*SeMalt*4, *SeMalt*4/*SeMalt3*/*SeMalt*1/*SeMalt*4, *SeMalt*4/*SeMalt*3/*SeMalt*2/*SeMalt*4, or other combinations of these transporters.

A further preferred mutant *S. eubayanus* yeast according to the invention has a chimeric maltose transporter gene comprising a N-terminal part and a C-terminal part of *Se*Malt2 and/or *Se*Malt4.

A most preferred mutant *S. eubayanus* yeast according to the invention has a chimeric maltose transporter gene comprising *Se*Malt4/*Se*Malt1/*Se*Malt2 or *Se*Malt4/*Se*Malt3. Said most preferred chimeric gene preferably comprises nucleotides 1-434 of *SeMALT4,* nucleotides 430-1122 of *SeMALT1*, nucleotides 1113-1145 of *SeMALT2* or *SeMALT4,* and nucleotides 1141-1842 of *SeMALT3,* as depicted in Figure 3.

Further genes that are preferably altered, preferably by random mutagenesis, are genes involved in decarboxylation activity of phenolic acids, preferably in producing 4-vinyl guaiacol, more preferably in decarboxylating ferulic acid into 4-vinyl guaiacol. Fermented beverages wherein phenolic compounds are generally considered as off flavors include beer, more preferably a beer selected from the group consisting of lager, wild lager, pilsner, pale ale and saison.

In beers, some of the phenolic (off-)flavors originate directly from the wort, others are a result of the enzymatic conversion by yeast, or through chemical conversion as a consequence of oxygen and temperature (e.g. during wort boiling or ageing in the bottle). During beer fermentation, ferulic acid that is present in the wort is converted through enzymatic decarboxylation into the phenolic off-flavor 4-VG. Initially only *PAD1,* encoding a phenylacrylic acid decarboxylase, was thought to be involved, but results from Mukai et al. (Mukai et al., 2010. J Bioscie Bioeng 109: 564-569) suggest that both *PAD1* and *FDC1,* encoding a ferulic acid decarboxylase, are necessary for decarboxylation. Top fermenting yeasts generally contain an active set of *PAD1* and *FDC1,* while bottom fermenting yeasts are not able to convert the phenolic acids into the corresponding phenolic off-flavors.

A preferred fermentative yeast comprises a mutation in at least one of the genes *PAD1* and *FDC1* and/or a gene involved in transcriptional control of at least one of said genes, and/or a gene encoding a protein involved in uptake of a phenolic acid, preferably ferulic acid, or involved in export of a decarboxylated phenolic compound, preferably 4-vinyl guaiacol, and/or a gene involved in transcriptional control of said gene.

Said phenolic acid preferably is a phenolic acid that can be converted by a protein encoded by *PAD1* and/or a protein encoded by *FDC1,* more preferably selected from ferulic acid, 4 hydroxy benzoate, sinapic acid, caffeic acid, cinnamic acid, 3,4-dihydroxybenzoic acid, ferulic acid, gallic acid, p-coumaric acid, 4-methoxycinnamic acid, p-hydroxybenzoic acid, 4-hydroxybenzaldehyde, protocatechuic acid, salicylic acid, syringic acid, tannic acid and/or vanillic acid. A particularly preferred substrate is ferulic acid, the uptake of which preferably is reduced or even inhibited in a preferred fermentative yeast that is used in the methods of the invention.

Examples of proteins involved in the export of a product of a protein encoded by *PAD1* and/or a protein encoded by *FDC1* is Pdr16 / YNL231C, Pdr8 / YLR266C, Pdr12 / YPL058C, Pdr10 / YOR328W, Pdr5 / YOR153W, Pdr18 / YNR070W, Pdr3 / YBL005W, Pdr15 / YDR406W, Pdr17 / YNL264C and Pdr11 / YIL013C. Said product is preferably a decarboxylated phenolic compound, more preferably 4-VG, 4-vinylphenol, 4 ethyl phenol, guaiacol and eugenol. A particularly preferred product is 4-VG.

The invention therefore provides a mutant *S. eubayanus* yeast according to the invention, which has a reduced decarboxylation activity of phenolic acids, preferably is not producing 4-vinyl guaiacol.

A preferred mutant *S. eubayanus* yeast according to the invention may in addition comprise one or more genomic alterations selected from a duplication of the right arm of Chromosome VIII, an alteration in the transcription factor gene *SEF1,* an alteration in the palmitoyl transferase gene *AKR1*, which is involved in endocytosis and cell shape control, an alteration in repressor of the glucose sensing signal pathway Mth1, an alteration in the gene encoding Bypass of Stop Codon protein 1 (Bsc1), which is a protein of unconfirmed function; similar to cell surface flocculin Flo11p, an alteration in the ammonium permease regulating gene *PAR32*, an alteration in the negative regulator of sporulation Mds3, an alteration in ergosterol biosynthesis gene Erg25, an alteration in the gene encoding a transcription factor involved in starvation response *ZPR1*, an alteration in the gene encoding a transcription factor involved in starvation response *STP2*, an alteration in the gene encoding a protein required for fermentation at low temperature *CSF1,* an alteration in the gene encoding a protein involved in regulation of sterol biosynthesis *NSG1.*

Said additional one or more genomic alterations preferably include a L895P alteration in the transcription factor gene *SEF1*; a S50P alteration in the palmitoyl transferase gene *AKR1*; an alteration in the DNA promoter region of *MTH1*, preferably a G(-753)A alteration; a L469S alteration in *BSC1*; an alteration in the DNA promoter region of *PAR32*, preferably G(-1266)A, G(-1265)A, G(-1237)A and/or G(-1236A); a P727H alteration in *MDS3*; a an alteration in the nucleic acid Terminator region of *ERG25*, preferably a G(+165)A alteration; an alteration in one or both zinc-finger domains (AA 52-210 and 293-486) of *ZPR1*, preferably a S327P alteration; a S181L alteration in the transcription factor *STP2*; a S2708P alteration in CSF1; and/or a G163A alteration in *NSG1.*

Unless otherwise indicated, the alterations refer to an amino acid alteration of the first named single letter amino acid residue for the second named single letter amino acid residue at the indicated position. For example, a S50P alteration in *AKR1* refers to the exchange of a serine at position 50 for a proline in *AKR1.* The positions in the nucleic acid promoter and Terminator regions refer to the nucleotide position relative to the transcription start or relative to the stop codon, respectively.

### 4.4 Hybrid yeast, generated from mutant S. eubayanus yeast

The invention furthermore provides a method for producing a hybrid yeast, comprising a) providing the mutant *S. eubayanus* yeast according to the invention as a first parent, and a second yeast as a second parent, which said second parent differs from the first parent, b) hybridizing cells from the first parent with cells from the second parent and c) identifying a resulting hybrid organism.

*Saccharomyces* hybrids are most commonly found in domesticated environments and are used in various industrial fermentation processes [Boynton and Greig, 2014. Yeast, 31: 449-462; Gorter de Vries et al., 2017. Applied Environm Microbiol 83: e03206-16]. For instance, lager brewing is performed with *S. pastorianus,* a hybrid between *S. cerevisiae* and *S. eubayanus* [Libkind et al., 2011. PNAS 108: 14539-14544], which combines the fermentative capacity and sugar utilisation of *S. cerevisiae* with the cryotolerance of *S. eubayanus* [Hebly et al., 2015. FEMS Yeast Res 15: fov005]. Various double and triple hybrids between *S. cerevisiae, S. kudriavzevii* and *S. uvarum* have been isolated from wine fermentations and appear to play an important role in aroma production [Gonzalez et al., 2006. FEMS Yeast Res 6: 1221-1234] .

Said second parent preferably is a yeast of the *Saccharomyces* sensu stricto complex. A more preferred yeast is a *Saccharomyces cerevisiae* yeast, a *S.* *carlsbergensis* yeast, a *S. pastorianus* yeast, a *S. eubayanus* yeast, and/or a hybrid thereof, preferably a *S. cerevisiae* yeast.

The combination of two or more *Saccharomyces* genomes in a hybrid commonly results in synergistic effects, a phenomenon called 'heterosis' or 'hybrid vigor', which enables the hybrid to perform better than either of its parents in specific environments [Shapira et al., 2014. Heredity 113: 316]. Therefore, targeted hybridisation of *Saccharomyces* yeasts is commonly used to generate strains with new or improved phenotypes for industrial applications. For instance, laboratory-made *S*. *cerevisiae* × *S. eubayanus* hybrids showed higher cold tolerance and oligosaccharide consumption [Hebly et al., 2015. FEMS Yeast Res 15: fov005], different flavour profiles [Steensels et al., 2014. Applied Environment Microbiol 80: 6965-6975], higher fermentation rates and higher ethanol titers [Krogerus et al., 2015. J Industrial Microbiol & Biotechnol 42: 769-778] than their parental strains.

Heterosis is a complex phenomenon which is not yet fully understood; it is most likely caused by a combination of multiple factors, including the amount of chromosomal copy numbers [Gorter de Vries et al., 2017. Applied Environm Microbiol 83: e03206-16; Krogerus et al., 2016. Appl Microbiol Biotechnol 100: 7203-7222], interactions between different dominant and recessive alleles and epistatic interactions [Shapira et al., 2014. Heredity 113: 316]. The resulting phenotype is not always ambiguous: dominant and usually more complex phenotypes such as cryotolerance or flocculation are usually completely inherited from one of the parental strains [Hebly et al., 2015. FEMS Yeast Res 15: fov005; Coloretti et al., 2006. Food Microbiol 23: 672-676], while for flavour compounds and other secondary metabolites the hybrids generally produce concentrations around the average of the concentrations produced by their parental strains [Krogerus et al., 2015. J Industrial Microbiol & Biotechnol 42: 769-778; Bellon et al., 2011. Appl Microbiol and Biotechnol 91: 603-612]. Heterosis is not only dependent on the parental species used for interspecies hybridization, but also on the specific strains used, making it even more difficult to predict the phenotype of an outcross.

Interspecies hybrids of species without a prezygotic barrier can be obtained analogously to intraspecific mating: hybrids are formed by either mating haploid strains of opposite mating type, or by rare mating between strains which do not have opposite mating types that have undergone spontaneous loss of heterozygosity in the mating type locus [Steensels et al., 2014. FEMS Microbiol Reviews 38: 947-995]. Interspecies hybridization has a relatively low occurrence rate; hybridization frequencies are reported to range from 1.5 - 3.6 % for spore-to-spore mating [Krogerus et al., 2016. Appl Microbiol Biotechnol 100: 7203-7222; Mertens et al., 2015. Appl Environment Microbiol 81: 8202-8214] to frequencies as low as 1 × 10-6 to 1 × 10-7 for rare mating [Krogerus et al., 2017. Microbial Cell Factories 16: 66; Gunge and Nakatomi, 1972. Genetics 70: 41-58].

In order to enhance identify the hybrid products, especially of rare mating events, a preferred method for producing a hybrid yeast comprises labeling of cells from the first and/or second parent with a fluorescent dye, prior to hybridizing the cells.

Cells of a first parent yeast may be labeled with a first dye, herein after termed dye A, while cells of a second parent yeast may be labeled with a second dye, herein after termed dye B. Dye A and dye B are fluorescent dyes, whereby dye A differs from dye B. In addition, cells labelled with dye A preferably can be distinguished from cells labelled with dye B; for example by employing dyes with different excitation and/or emission spectra. Suitable dyes that can be used in methods of the invention can be excited by a monochromatic light source, preferably a laser, more preferably by an ultraviolet laser (about 355 nm), a violet laser (about 405 nm), a blue laser (about 488 nm) or a red laser (about 640 nm). For example, dye A may be a dye that is excited with a red laser at about 630 nm, and which emits at about 661 nm, while dye B is a dye that is excited with a blue laser at about 492 nm and which emits at about 517 nm.

Labelling preferably is direct. Labelling is preferably preformed by labelling primary amines (R-NH2) of proteins, amine-modified oligonucleotides, and other amine-containing molecules.

For this, a dye preferably comprises a succinimidyl group, preferably a succinimidyl ester, to couple the dye to intracellular lysine residues and other amine sources.

Further preferred dyes include thiol-reactive dyes, in which a fluorescent label is coupled to, for example, iodoacetamide, maleimide, benzylic halide or a bromomethylketone, In addition, microinjectable dyes comprising a polar dye such as lucifer yellow CH, Cascade Blue hydrazide, Alexa Fluor hydrazides and biocytin that may be introduced into a cell by whole-cell patch clamping, iontophoresis, osmotic lysis of pinocytic vesicles; and/or fluorescent dextran conjugates or fluorescent microspheres that may be loaded into cells by invasive techniques such as microinjection, whole-cell patch clamping, scrape loading, microprojectile bombardment, electroporation or osmotic shock, can be used to stain cells in methods of the invention.

Said fluorescent label preferably is selected from Abz (Anthranilyl, 2-Aminobenzoyl), N-Me-Abz (N-Methyl-anthranilyl, N-Methyl-2-Aminobenzoyl), FITC (Fluorescein isothiocyanate), 5-FAM (5-carboxyfluorescein), 6-FAM (6-carboxyfluorescein), TAMRA (carboxytetramethyl rhodamine), Mca (7-Methoxycoumarinyl-4-acetyl), AMCA or Amc (Aminomethylcoumarin Acetate), Dansyl (5-(Dimethylamino) naphthalene-1-sulfonyl), EDANS (5-[(2-Aminoethyl)amino] naphthalene-1-sulfonic acid), Atto (e.g. Atto465, Atto488, Atto495, Atto550, Atto647), cyanine (Cy) dyes, including Cy3 (1-(5-carboxypentyl)-3,3-dimethyl-2-((1E,3E)-3-(1,3,3-trimethylindolin-2-ylidene)prop-1-en-1-yl)-3H-indol-1-ium chloride), Cy5 (1-(5-carboxypentyl)-3,3-dimethyl-2-((1E,3E,5E)-5-(1,3,3-trimethylindolin-2-ylidene)penta-1,3-dienyl)-3H-indolium chloride), including trisulfonated Cy5, and Cy7 (1-(5-carboxypentyl)-2-[7-(1-ethyl-5-sulfo-1,3-dihydro-2H-indol-2-ylidene)hepta-1,3,5-trien-1-yl]-3H-indolium-5-sulfonate), Alexa Fluor (e.g. Alexa Fluor 647, Alexa488, Alexa532, Alexa546, Alexa594, Alexa633, Alexa647), Bodipy (e.g. Bodipy® FL), Dylight (e.g. DyLight 488, DyLight 550), Lucifer Yellow (ethylene diamine or 6-amino-2-(2-amino-ethyl)-1,3-dioxo-2,3-dihydro-1H-benzo[de]isoquinoline-5,8-disulfonic acid) and derivatives thereof.

It will be clear to a person skilled in the art that preferred dye combinations include dyes that can be distinctly measured, preferably by two emission filters without spectral overlap, preferably without the need for fluorescence compensation, more preferably dyes that can be excited by two different by two different lasers to minimize spectral overlap, such as with a violet laser (about 405 nm), a blue laser (about 488 nm) or a red laser (about 640 nm). Preferred combinations, which allows cells that are stained with dye A to identify and isolate from cells stained with dye B are fluorescent dyes that can be excited with a violet laser and a blue laser; with a violet laser and a red laser, or with a blue laser and a red laser.

Dye A and dye B preferably are dyes that also allow the identification and isolation of cells that harbor both dye A and dye B, from cells that harbor only dye A and only dye B. For this, preferred dyes include a dye that is excited with a red laser at about 630 nm, and which emits at about 661 nm, and a dye that is excited with a blue laser at about 492 nm and which emits at about 517 nm.

Hybridization of labeled cells is preferably performed in the dark to prevent bleaching of the fluorescent dyes, as will be clear to a person skilled in the art.

In order to enhance the identification of hybrid products, especially of rare mating events, the hybridization of labeled or unlabeled parent yeast cells preferably is performed at a temperature that is at least 5 °C below the optimal growth temperature of the first and/or the second parent yeast.

Hybridization preferably is performed at a temperature that is below the optimal growth temperature of both parent yeasts, in order to prevent excessive cell proliferation. By reducing the temperature, cell division takes longer, while hybridization is less affected. Hence, a higher proportion of the resulting cells are hybrid cells, when compared to hybridization at a higher temperature. A hybridization temperature that is at least 5 °C below the optimal growth temperature of the parent yeasts was found to limit loss of staining by the dyes and to result in identification of rare interspecies hybrids resulting from hybridization between the first parent yeast and the second parent yeast.

A temperature that is at least 5 °C below the optimal growth temperature of the first and/or the second parent organism is preferably below 18 °C, preferably between 5 °C and 15 °C, more preferably between 10 °C and 13 °C, most preferably about 12 °C. A person skilled in the art is unquestionably able to determine an optimal growth temperature of a yeast with an unusual optimal growth temperature, for example by growing cells of the yeast at different temperatures.

The invention further is directed to a hybrid yeast comprising a copy of the genome of a mutant *Saccharomyces eubayanus* yeast that is able to ferment maltotriose. Said hybrid yeast preferably is produced by a method for producing a hybrid yeast according to the invention.

### 4.5 Methods of producing a fermented beer product

Yeasts have been used since long in baking, brewing and distilling, such as in bread production and beer and wine fermentation.

Brewer's wort comprises fermentable sugars including maltose (50-60%), maltotriose (15-20%) and glucose (10-15%). The methods of the invention preferably employ a mutant *S. eubayanus* yeast, and/or a hybrid thereof, that is that is able to ferment maltotriose. The use of a mutant *Saccharomyces eubayanus* yeast that is able to ferment maltotriose according to the invention, and/or a hybrid yeast according to the invention, will influence the organoleptic characteristics of the resulting fermented beer product.

Said yeast may further comprise one or more naturally occurring mutations, and/or mutations resulting from mutagenesis, in at least one of the genes *PAD1* and *FDC1,* a gene involved in transcriptional control of at least one of said genes, and/or a gene encoding a protein involved in uptake of a phenolic acid, preferably ferulic acid, or involved in export of a decarboxylated phenolic compound, preferably 4-vinyl guaiacol, and/or a gene involved in transcriptional control of said gene.

Said method for producing a fermented beer product comprises the provision of mashed cereal grains, preferably barley, in an aqueous solution, preferably in water, to release the malt sugars. This malting step is followed by boiling the resulting wort in the presence of hop, and fermenting the resulting boiled wort after cooling. When fermentation is completed, the beer may be filtered and bottled.

During the fermentation process, fermentable sugars are converted into alcohols such as ethanol, CO₂ and flavor compounds such as esters, for example isoamyl acetate. As is known to a person skilled in the art, factors that will influence the appearance and taste of the resulting product include, but are not limited to, roasting temperature and roasting time of the grains, temperature and time of steeping, germination, and kilning of the grains, temperature and time of milling and mashing of the grains, lautering of the resulting mash to generate the wort, temperature and time of boiling of the wort, timing and amounts of added hop, the specific hop that is used, temperature and time of fermentation, type of yeast, mechanically filtering of the yeast or the addition of filtering agents to remove the yeast and finally, carbonating and packaging of the beer. During a conditioning step, which may start after fermentation but before filtering, the yeast is given time, from days to weeks, to absorb common off flavors associated with under-conditioned or "green" beer, including sulfur, butter, and green apples.

In the methods of the invention, the fermentation process is performed at normal temperatures, preferably 6-25 °C, preferably 7-20 °C, more preferably 8-13 °C, including. Lager beer fermentation is generally performed at temperatures between 7-13 °C.

In one embodiment, the amount of alcohol is reduced after fermentation. To reduce the amount of alcohol in the final beer product, the resulting beer product with an alcohol concentration above 4 vol % is subjected to a physical process involving, for example, rectification and/or dialysis, including reverse osmosis.

Rectification is usually performed under reduced pressure to achieve boiling of the volatile ethanol at a temperature that does not result in breakdown of other ingredients such as proteins and sugars. Said rectification preferably is performed after fermentation at an elevated temperature at 20-50 °C under reduced pressure. Methods for vacuum rectification to reduce alcohol levels have been described, e.g. by Narziss et al., 1993. Brauwelt 133: 1806-1820, and Kern 1994. Alimentacion Equipos y Tecnologia 13: 37-41. Further suitable methods include falling film rectification (Zufall and Wackerbauer, 2000. Monatsschrift fuer Brauwissenschaft 53: 124-137). Suitable large scale rectification systems are available from, for example, KmX Chemical Corporation, New Church, Virginia, Pope Scientific, Inc., Saukville, Wisconsin, M&L Engineering GmbH, Hofheim am Taunus, Germany, Centec, Maintal, Germany, and API Schmidt Bretten GmbH & Co. KG, Bretten, Germany.

Dialysis to reduce alcohol content of a fermented beverage includes passaging of the beverage through a semi-permeable membrane (German Pat. Nos. 2 145 298 and 2 413 236). A preferred dialysis process is a single reverse osmosis process to separate a beverage into a concentrate and a filtrate (Belgian Pat. No. 717 847, German Pat. No. 2 323 094, German Pat. No. 2 339 206). Further variants comprise comprising reverse osmosis (U.S. Pat. No. 4,317,217) and pervaporation (European Patent Application 332,738). The threshold features of the membrane used determines which low molecular weight molecules, such as the salts, esters and aldehydes, are removed together with the alcohol from the fermented beverage. In addition, the high pressure that is exerted during the process may cause denaturation of molecules, resulting in alterations in physical-chemical properties, such as increased turbidity, flocculation, etc., and in organoleptic properties such as modified flavor and taste. Suitable large scale dialysis systems are available from, for example, Alfa Laval, Lund, Sweden and Osmonics Inc., Minnetonka, Minnesota.

### 5. EXAMPLES

### Example 1

### Materials and methods

### Strains and maintenance

*S. eubayanus* type strain CBS 12357 (Libkind et al., 2011. Proc Natl Acad Sci U S A 108: 14539-44), was obtained from the Westerdijk Fungal Biodiversity Institute (Utrecht, the Netherlands). All strains used in this study are listed in Tables 1 and 3. Stock cultures of *S. eubayanus* strains were grown in YPD (10 g L-1 yeast extract, 20 g L-1 peptone and 20 g L-1 glucose) until late exponential phase, complemented with sterile glycerol to a final concentration of 30% (v/v) and stored at -80 °C until further use.

### Media and cultivation

Plasmids were propagated overnight in Escherichia coli XL1-Blue cells in 10 mL LB medium containing 10 g L-1 peptone, 5 g L-1 Bacto Yeast extract, 5 g L-1 NaCl and 100 mg L-1 ampicillin at 37 °C. Synthetic medium (SM) contained 3.0 g L-1 KH2PO4, 5.0 g L-1 (NH4)2SO4, 0.5 g L-1 MgSO4, 7 H2O, 1 mL L-1 trace element solution, and 1 mL L-1 vitamin solution (Verduyn et al., 1992. Yeast 8: 501-17), and was supplemented with 20 g L-1 glucose (SMG), maltose (SMM) or maltotriose (SMMt) by addition of an autoclaved 50% solution. Maltotriose with a purity of 95,8% was used (Glentham Life Sciences, Corsham, United Kingdom). Industrial wort was provided by HEINEKEN Supply Chain B.V., Zoeterwoude, the Netherlands. The wort was supplemented with 1.5 g L-1 of Zn2+ by the addition of Zinc heptahydrate sulfate, autoclaved for 30 minutes at 121°C and filtered using Nalgene 0.2 µm SFCA bottle top filters (Thermo Scientific) prior use. For experiments performed with diluted wort, sterile demi water was added to the filtered wort in the appropriate volume. Aerobic cultures were grown in 500 mL shake flasks with 100 mL medium. For cultivation on solid media, media were supplemented with 20 g L-1 of agar. Shake flask and bottle cultures were incubated at 200 RPM in a New Brunswick Innova43/43R shaker (Eppendorf Nederland B.V., Nijmegen, The Netherlands). Selection of the *S*. *eubayanus* strains transformed with plasmids pUDP052 (gRNASeSGA1) was carried out on a SMAceG: SMG medium in which (NH4)2SO4 was replaced by 5 g L-1 K2SO4 and 10 mM acetamide as described previously (Solis-Escalante et al., 2013. FEMS Yeast Res 13: 126-39).

### Aerobic shake flask cultivations

Aerobic shake flask cultivations were inoculated from stationary phase aerobic precultures. Growth studies on SMMt and SMM were pre-cultured on SMM, growth studies on SMG were pre-cultured on SMG and growth studies on three-fold diluted wort were pre-cultured on three-fold diluted wort. Growth experiments were performed in 500 ml shake flasks containing 100 ml of medium and were inoculated to an OD₆₆₀ of 0.1. The shake flasks were incubated at 20 °C and 200 RPM and samples were taken at regular intervals to determine extracellular metabolite concentrations.

### Microaerobic bottle characterization

Bottle cultivations were performed in 250 mL airlock-capped bottles, with a working volume of 200 mL on *threefold diluted wort* supplemented with 0.4 mL L-1 pluronic to prevent foaming (Sigma-Aldrich). The membrane of the lid was equipped with a short needle capped with a 0.2 µm filter to prevent pressure build-up and sampling was performed aseptically through a needle with a 3 mL syringe. The bottles were inoculated to an OD₆₆₀ of 0.1 from stationary phase precultures in 50 ml aerobic Greiner reactor tubes containing 30 ml of the same medium after 4 days of incubation at 12 °C. The bottles were incubated at 12 °C and 200 RPM, and 3,5 mL samples were collected in a 24 deep well plate using a liquid handler LiHa (Tecan, Männedorf, Switzerland) at regular intervals to measure OD₆₆₀ and external metabolites. For each sample, 30µL was 5 times diluted to 150 µL in a 96 well plate and the OD₆₆₀ was measured using a Magellan Infinite 200 PRO spectrophotometer (Tecan, Männedorf, Switzerland), and the remaining sample was filter sterilized for HPLC measurements.

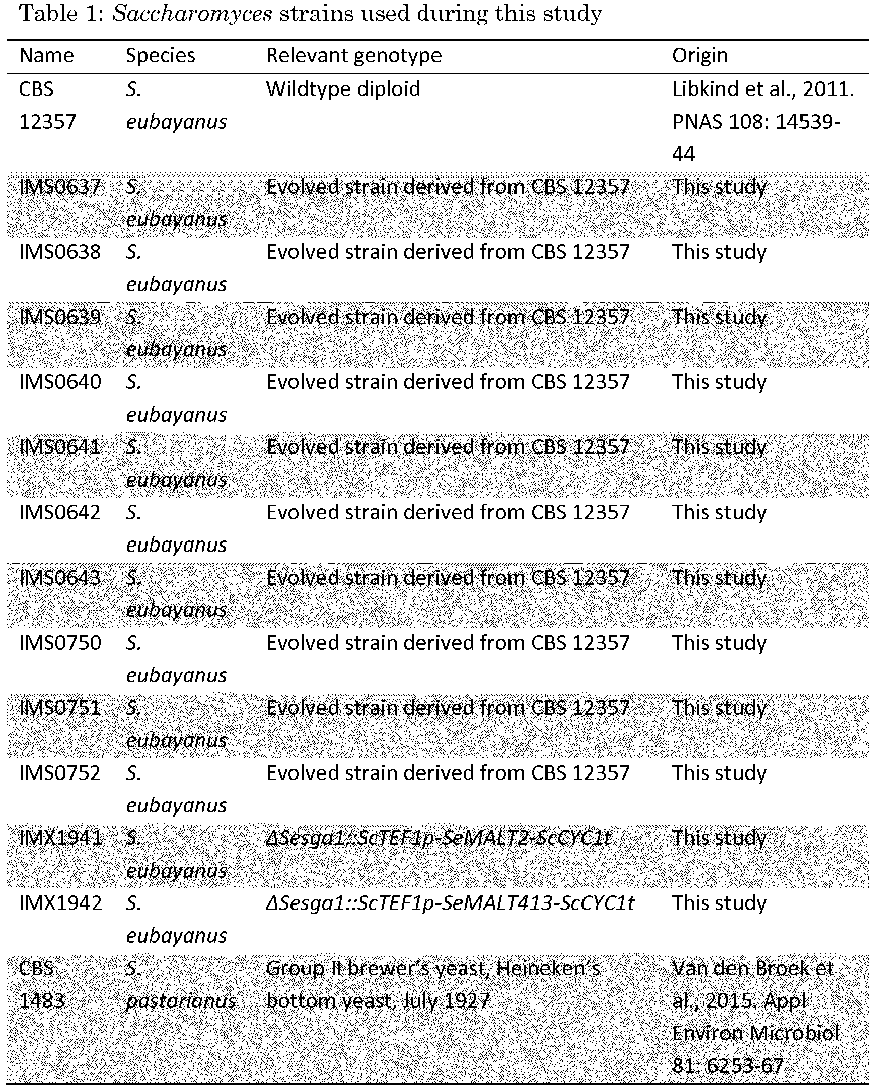

### UV mutagenesis and selection

CBS 12357 was grown aerobically on SMG at 20 °C until stationary phase and diluted to an OD₆₆₀ of 1.0 with milliQ. 50 mL of this solution was spun down at 4816 g for 5 minutes and resuspended in milliQ water twice. 25 ml of washed cells was poured into a 100 mm x 15 mm petri dish without lid, and irradiated with a UV lamp (TUV 30 W T8, Philips, Eindhoven, The Netherlands) at a radiation peak of 253,7 nm. 25 mL of non-mutagenized and 5 mL of mutagenized cells were kept to determine the survival rate. From both samples a 100-fold dilution was made, from which successive 10 fold dilutions were made down to a 100,000-fold dilution. Then, 100 µL of each dilution was plated on YPD agar and the number of colonies was counted after incubation during 48h at room temperatures. After 10,000 fold dilution, 182 colonies formed from the non-mutagenized cells against 84 colonies for the mutagenized cells, indicating a survival rate of 46%. The remaining 20 ml of mutagenized cells was spun down at 4816 g for 5 minutes and resuspended in 1 ml milliQ water. The mutagenized cells were added to a 50 mL shake flask containing 9 mL SMMt and incubated for 21 days at 20°C and 200 RPM. Maltotriose concentration was recorded at day 0, 19 and 21. On the 21st day, 100 µL of grown culture was transferred twice to a fresh shake flask with SMMt and incubated until stationary phase. At the end of the second transfer, single cell isolates were obtained using the BD FACSAria™ II SORP Cell Sorter (BD Biosciences, Franklin Lakes, NJ) equipped with 355 nm, 445 nm, 488 nm, 561 nm and 640 nm lasers and a 70 µm nozzle, and operated with filtered FACSFlow™ (BD Biosciences). Cytometer performance was evaluated prior to each experiment by running a CST cycle with CS&T Beads (BD Biosciences). Drop delay for sorting was determined by running an Auto Drop Delay cycle with Accudrop Beads (BD Biosciences). Cell morphology was analysed by plotting forward scatter (FSC) against side scatter (SSC). Gated single cells were sorted into a 96-well microtiter plates containing SMMt using a "single cell" sorting mask, corresponding to a yield mask of 0, a purity mask of 32 and a phase mask of 16. The 96 well plate was incubated during 96h at room temperature in a GENIos Pro micro plate spectrophotometer (Tecan, Männedorf, Switzerland) and growth was monitored by OD₆₆₀. Finally, the biomass was resuspended and the final OD₆₆₀ was measured. The 7 isolates with the highest final OD₆₆₀ were picked, restreaked and stocked as IMS0637-643.

### Maltotriose-limited chemostat cultivation

Chemostat cultivations were performed in Multifors 2 Mini Fermenters (INFORS HT, Velp, The Netherlands) equipped with a level sensor to maintain a constant working volume of 100 mL. The culture temperature was controlled at 20 °C and the dilution rate was set at 0.03 h⁻¹ by controlling the medium inflow rate. Cultures were grown on six-fold diluted wort supplemented with 10 g L⁻¹ additional maltotriose (Glentham Life Sciences), 0,2 mL L⁻¹ anti-foam emulsion C (Sigma-Aldrich, Zwijndrecht, the Netherlands), 10 mg L⁻¹ ergosterol, 420 mg L⁻¹ TWEEN® 80 and 5 g L⁻¹ ammonium sulfate. The TWEEN® and ergosterol were added as a solution as described previously (Verduyn et al., 1992. Yeast 8: 501-17). IMS0637-IMS0643 were grown overnight at 20 °C and 200 RPM in separate shake flasks on three-fold diluted wort. The OD₆₆₀ of each strain was measured and the equivalent of 7 mL at an OD₆₆₀ of 20 from each strain was pooled in a total volume of 50 mL. To inoculate the reactor, 20 mL of the pooled cultures was used. After overnight growth, the medium inflow pumps were turned on and the fermenter was sparged with 20 mL min⁻¹ of nitrogen gas and stirred at 500 RPM. The pH was not adjusted. Samples were taken weekly. Due to a technical failure on the 63rd day, the chemostat was autoclaved, cleaned and restarted using a sample taken on the same day. After a total of 122 days, the chemostat was stopped and single colony isolates were sorted onto SMMt agar using the FACS, as for IMS0637-IMS0643. Three colonies were randomly picked, restreaked and stocked as IMS0750-752.

### Genomic isolation and whole genome sequencing

Yeast cultures were incubated in 50-ml Greiner tubes containing liquid YPD medium at 20°C on an orbital shaker set at 200 RPM until the strains reached stationary phase with an OD₆₆₀ between 12 and 20. Genomic DNA for whole genome sequencing was isolated using the Qiagen 100/G kit (Qiagen, Hilden, Germany) according to the manufacturer's instructions and quantified using a Qubit® Fluorometer 2.0 (ThermoFisher Scientific, Waltham, MA).

Genomic DNA of the strains CBS 12357, IMS0637-IMS0643 and IMS0750-IMS0752 was sequenced by Novogene Bioinformatics Technology Co., Ltd (Yuen Long, Hong Kong) on a HiSeq2500 sequencer (Illumina, San Diego, CA) with 150 bp paired-end reads using PCR-free library preparation. All reads are available at NCBI (https://www.ncbi.nlm.nih.gov) under the bioproject accession number PRJNA492251.

Genomic DNA of strains IMS0637 and IMS0750 was sequenced on a Nanopore MinION (Oxford Nanopore Technologies, Oxford, United Kingdom). Libraries were prepared using 1D-ligation (SQK-LSK108) as described previously (Salazar et al., 2017. FEMS Yeast Res 17: doi: 10.1093/femsyr/fox074) and analysed on FLO-MIN106 (R9.4) flow cell connected to a MinION MklB unit (Oxford Nanopore Technology). MinKNOW software (version 1.5.12; Oxford Nanopore Technology) was used for quality control of active pores and for sequencing. Raw files generated by MinKNOW were base called using Albacore (version 1.1.0; Oxford Nanopore Technology). Reads with a minimum length of 1000 bp were extracted in fastq format. All reads are available at NCBI (https://www.ncbi.nlm.nih.gov/) under the bioproject accession number PRJNA492251.

### Genome analysis

For the strains CBS 12357, IMS0637-IMS0643, IMS0750-IMS0752 and IMS0760-IMS0762, the raw Illumina reads were aligned against a chromosome-level reference genome of *S. eubayanus* type strain CBS 12357 (Brickwedde et al., 2018. Front Microbiol 9: 1786) using the Burrows-Wheeler Alignment tool (BWA), and further processed using SAMtools and Pilon for variant calling (Li and Durbin, 2010. Bioinformatics 26:589-95; Li et al., 2009. Bioinformatics 25: 2078-9; Walker et al., 2014. PloS One 9: e112963). Heterozgous SNPs and INDELs which were heterozygous in CBS 12357 were disregarded. Chromosomal translocations were detected using Breakdancer (Chen et al., 2009. Nat Methods 6: 677). Only translocations which were supported by at least 10% of the reads aligned at that locus were considered. Chromosomal copy number variation was estimated using Magnolya (Nijkamp et al., 2012. Bioinformatics 28: 3195-202) with the gamma setting set to "none" and using the assembler ABySS (v 1.3.7) with a k-mer size of 29 (Simpson et al., 2009. Genome Res 19: 1117-23). All SNPs, INDELs, recombinations and copy number changes were manually confirmed by visualising the generated .bam files in the Integrative Genomics Viewer (IGV) software (Robinson et al., 2011. Nat Biotechnol 29: 24).

For strains IMS0637 and IMS0750, the nanopore sequencing reads were assembled *de novo* using Canu (version 1.3) (Koren et al., 2017. Genome Res 27: 722-736) with genome size set to 12 Mbp. Assembly correctness was assessed using Pilon (Walker et al., 2014. PloS One 9: e112963) and further correction "polishing" of sequencing/assembly errors was performed by aligning Illumina reads with BWA (Li and Durbin, 2010. Bioinformatics 26:589-95) using correction of only SNPs and short indels (-fix bases parameter). Long sequencing reads of IMS0637 and IMS0750 were aligned to the obtained reference genomes and to the reference genome of CBS 12357 using minimap2 (Li, 2018. Bioinformatics 34: 3094-3100). All reads are available at NCBI (https://www.ncbi.nlm.nih.gov/) under the bioproject accession number PRJNA492251.

### Molecular biology methods

For colony PCR and Sanger sequencing, genomic DNA was prepared by boiling in 10 µL 0.02 M NaOH for five minutes. To verify isolates belonged to the *S*. *eubayanus* species, the presence of *S. eubayanus*-specific gene SeFSY1 was tested by PCR amplification using primers 8572 and 8573 (Pengelly and Wheals, 2013. FEMS Yeast Res 13: 156-61), and the absence of *S. cerevisiae*-specific gene ScMEX67 was tested by PCR amplification using primers 8570 and 8571 (Muir et al., 2011. FEMS Yeast Res 11: 552-63). For further confirmation of *S. eubayanus* nature, the ITS regions were amplified using primers 10199 and 10202 and the amplified fragments were Sanger sequenced (Schoch et al., 2012. Proc Natl Acad Sci USA 109: 6241-6). Resulting sequences were compared to available ITS sequences and classified as the species to which the amplified region had the highest sequence identity. The presence of the SeMALT genes was verified by PCR using gene specific primers: 10491 and 10492 for SeMALT1, 10632 and 10633 for SeMALT2 and SeMALT4/2, 10671 and 10672 for SeMALT3, 10491 and 10671 for SeMALT13, and 10633 and 10671 for SeMALT413. The amplified fragments were gel-purified and Sanger sequenced using the primers used to amplify them.

### Plasmid construction

All plasmids and primers used in this study are listed in Table 2 and Table 3. DNA amplification for plasmid and strain construction was performed using Phusion High-Fidelity DNA polymerase (ThermoFisher Scientific) according to the supplier's instructions. The coding region of *SeMALT413* was amplified from genomic DNA of IMS0750 with primer pair 10633/10671. Each primer carried a 40 bp extension complementary to the plasmid backbone of p426-TEF-amds (Marques et al., 2017. FEMS Yeast Res 17:fox006), which was PCR amplified using primers 7812 and 5921. The fragment was "Gibson" assembled (Gibson et al., 2009. Nat Methods 6: 343) with the p426-TEF-amdS backbone fragment using NEBuilder HiFi DNA Assembly (New England Biolabs, Ipswich, MA), resulting in plasmid pUD814.

### Strain construction

To integrate and overexpress *SeMALT2* and *SeMALT413* ORFs in *S. eubayanus* CBS 12357, *SeMALT2* and *SeMALT413* were amplified from pUD480 and pUD814 respectively with the primers 13559/13560 that carried a 40 bp region homologous to each flank of the SeSGA1 gene located on *S. eubayanus* chromosome IX. To facilitate integration, the PCR fragments were co-transformed with the plasmid pUDP052 that expressed Spcas9^{D147YP411T} (Bao et al., 2014. ACS Synth Biol 4: 585-94; Gorter de Vries et al., 2017. Microb Cell Fact 16: 222) and a gRNA targeting *SeSGA1* (Brickwedde et al., 2018. Front Microbiol 9: 1786). The strain IMX1941 was constructed by transforming CBS 12357 with 1 µg of the amplified *SeMALT2* expression cassette and 500 ng of plasmid pUDP052 by electroporation as described previously (Gorter de Vries et al., 2017. Microb Cell Fact 16: 222). Transformants were selected on SMAceG plates. Similarly, IMX1942 was constructed by transforming CBS 12357 with 1 µg of the amplified *SeMALT413* expression cassette for *SeMALT413* instead of *SeMALT2.* Correct integration was verified by diagnostic PCR with primer pair 12635/12636. All PCR-amplified gene sequences were Sanger sequenced (Baseclear, Leiden, The Netherlands).

### Protein structure prediction

Homology modeling of the *Se*Malt413 transporter was performed using the SWISS-MODEL server (https://swissmodel.expasy.org/) (Biasini et al., 2014. Nucleic Acids Res 42(W1): W252-W8). SeMALT413 was translated and used as input. The model of the xylose proton symporter XylE (PDB: 4GBY) was chosen as template (Lam et al., 1980. J Bacteriol 143: 396-402). Models were built based on the target-template alignment using ProMod3. Coordinates which are conserved between the target and the template are copied from the template to the model. Insertions and deletions are remodeled using a fragment library. Side chains are then rebuilt. Finally, the geometry of the resulting model is regularized by using a force field. In case loop modelling with ProMod3 fails, an alternative model is built with PROMOD-II (Guex et al., 2009. Electrophoresis 30: S162-S73). 3D model was assessed and colored using Pymol (The PyMOL Molecular Graphics System, Version 2.1.1 Schrödinger, LLC.).

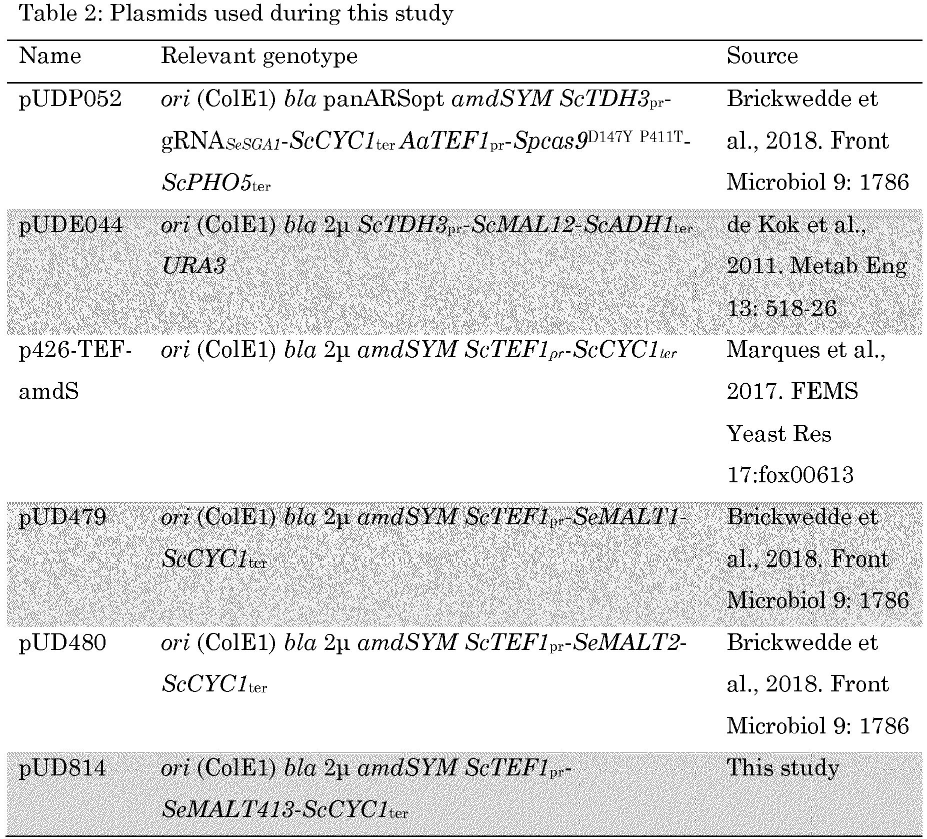

### Analytics

The concentrations of ethanol and of the sugars glucose, maltose and maltotriose were measured using a high pressure liquid chromatography (HPLC) Agilent Infinity 1260 series (Agilent Technologies, Santa Clara, CA) using a Bio-Rad Aminex HPX-87H column at 65°C and a mobile phase of 5mM sulfuric acid with a flow rate of 0,8 mL per minute. Compounds were measured using a RID at 35°C. Samples were spun down (13.000xg for 5 minutes) to collect supernatant or 0.2µm filter sterilized before analysis.

### Results

### Mutagenesis and evolution enables S. eubayanus to utilize maltotriose

The *Saccharomyces eubayanus* type strain CBS 12357 does consume maltose but not maltotriose, one of the main fermentable sugars in brewer's wort (Hebly et al., 2015. FEMS Yeast Res 15: fov005). In an attempt to obtain mutants able to utilize maltotriose, laboratory evolution was applied (see Figure 1). To increase the initial genetic diversity, the strain CBS 12357 was submitted to mild UV-mutagenesis, which resulted in a survival rate of 46%. The mutant pool was inoculated in SM medium containing 20 g L-1 maltotriose (SMMt) as the sole carbon source and incubated at 20 °C to enrich for maltotriose consuming mutants. After a lag phase of two weeks, growth was observed and the maltotriose concentration decreased to 10,48 g L-1 after 21 days. After two subsequent transfer in SMMt medium, 96 single cells were sorted into a microtiter YPD plate using FACS. Upon incubation, the resulting single-cell cultures were replica-plated into a microtiter SMMt plate and growth was monitored based on OD660. The seven isolates with the highest final OD660 were selected and named IMS0637-IMS0643. PCR amplification of the *S. eubayanus* specific SeFSY1 gene and sequencing of the ITS region confirmed that all 7 isolates belonged to the *S. eubayanus species* (data not shown). To characterize their growth on maltotriose, the wild type CBS 12357, the mutants IMS0637-IMS0643 and the maltotriose-consuming *S. pastorianus* strain CBS 1483 were characterized in shake flasks containing SMMt at 20 °C (Figure 2A). While CBS 12357 did not show any maltotriose consumption after 187 h, the residual amount of maltotriose dropped below 50% after 91 h for IMS0637-IMS0643 and after 43 h for CBS 1483. Despite the slower maltotriose utilization, the final maltotriose attenuation reached 92.7 ± 1.6% for IMS0637-IMS0643 mutants, comparably to the final attenuation of 92.1% reached

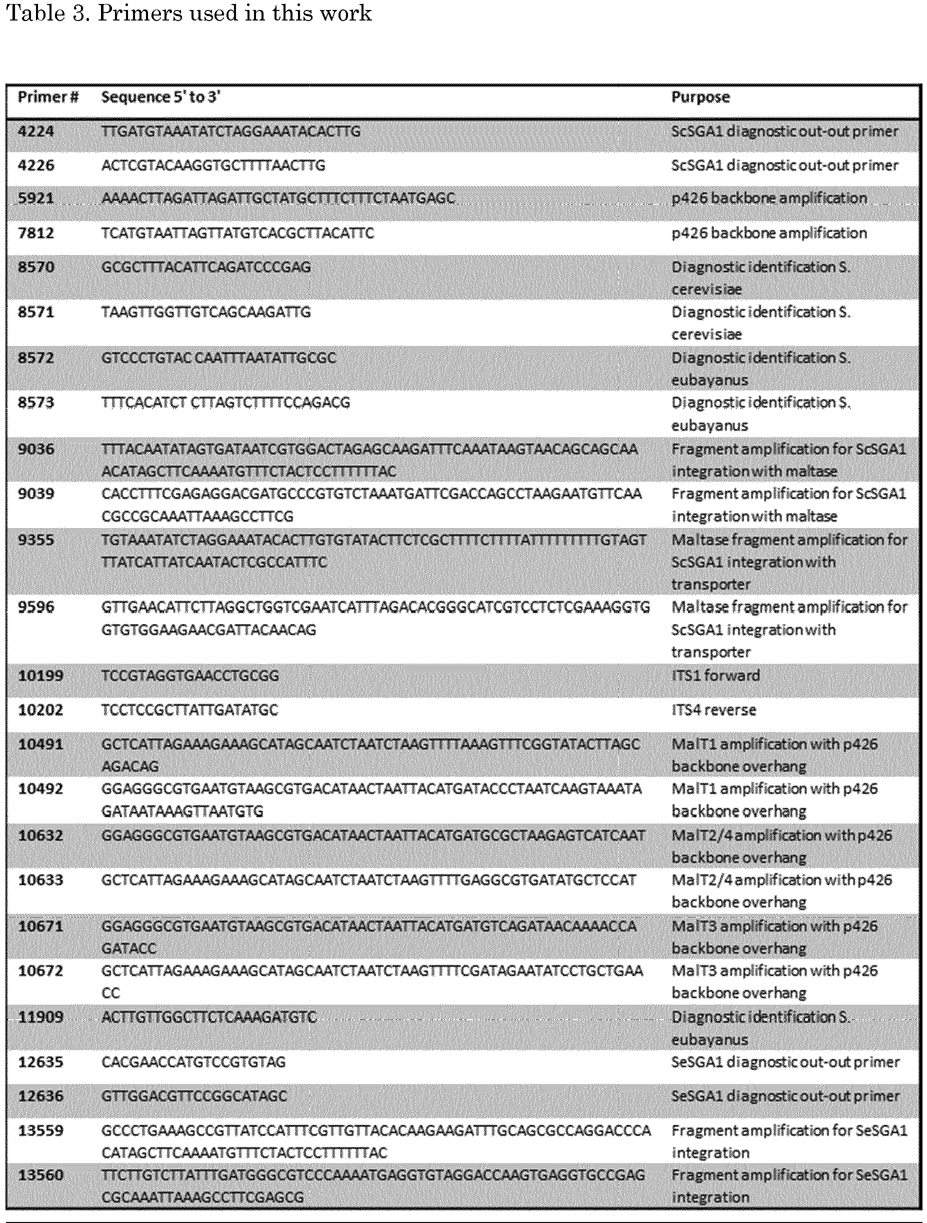

by the maltotriose consuming reference CBS 1483. While these results indicated that maltotriose was utilized in synthetic medium, maltotriose utilization in brewer's wort was needed for industrial applicability. The strains were characterized in shake flasks containing three-fold diluted wort. While *S. pastorianus* CBS 1483 had consumed 50% of the maltotriose after 145 h, the mutants IMS0637-IMS0643 had not consumed any maltotriose after 361 h, just as the *S. eubayanus* wild type CBS 12357 (Figure 2B). Therefore to improve the ability to utilize maltotriose under brewing conditions, the mutants IMS0637-IMS0643 were submitted to laboratory evolution in a carbon-limited chemostat on brewer's wort enriched with maltotriose. Under these conditions, mutants with an improved affinity or an higher transport rate for growth on maltotriose would be less nutrient-limited, resulting in a strong selective advantage. To this end, the cells were grown in six-fold diluted wort supplemented with 10 g L⁻¹ of maltotriose, resulting in a final concentration of 2 g L⁻¹ glucose, 15 g L⁻¹ maltose and 15 g L⁻¹ maltotriose. To prevent oxygen and nitrogen limitation, 10 mg L⁻¹ ergosterol, 420 mg L⁻¹ TWEEN® 80 and 5 g L⁻¹ ammonium sulfate were supplemented. The UV-mutants IMS0637-IMS0643 were pooled and used to inoculate the reactor. In the initial batch, all glucose and maltose was consumed, leaving maltotriose as the only carbon source left. The continuous culture was operated at a dilution rate of 0.03 h⁻¹, and the outflow initially contained 13.2 g L⁻¹ of maltotriose. Over a period of 121 days maltotriose concentration progressively decreased to 7.0 g L⁻¹ (Figure 2C). At that point, 10 single cells from the culture were FACS sorted on SMMt agar plates and incubated at 20 °C. PCR amplification of the *S. eubayanus* specific *SeFSY1* gene and sequencing of the ITS region confirmed that all tested isolates belonged to the *S. eubayanus* species (data not shown). Three single cell lines were isolated, named IMS0750, IMS0751 and IMS0752, and characterized at 12 °C in micro-aerobic cultures containing threefold diluted wort, along with the wild type CBS 12357 and the *S. pastorianus* CBS 1483 (Figure 2D). While CBS 12357 and IMS0751 were only able to consume glucose and maltose, the evolved isolates IMS0750, IMS0752 and CBS 1483 consumed maltotriose. After 263 h, the maltotriose concentration had decreased from 20 to 4.3 g L⁻¹ maltotriose for IMS0750 and IMS0752 and to 2.0 g L⁻¹ for CBS 1483. Due to its inability to utilize maltotriose in wort, IMS0751 was not studied further. These results confirmed that the evolved strains IMS0750 and IMS0752 were able to consume maltotriose in wort almost as well as the *S. pastorianus* reference CBS 1483, while the mutagenized strains IMS0637-IMS0643 were only able to utilize maltotriose when supplied as sole carbon source in synthetic medium (SMMt).

### Whole genome sequencing reveals a new recombined chimeric SeMALT gene

The genomes of wild type CBS 12357, of the UV-mutants IMS0637-IMS0643 and of the evolved strains IMS0750 and IMS0752 were sequenced using 150 bp paired-end Illumina reads. The sequencing data was mapped to a chromosome-level assembly of the genome of wild type CBS 12357 (Brickwedde et al., 2018. Front Microbiol 9: 1786) to identify SNPs, INDELs and CNV mutations relative to CBS 12357. The genomes of the UV-mutants IMS0637, IMS0640, IMS0641 and IMS0642 shared a set of 116 SNPs, 5 INDELs and 1 copy number variation (Figure 3A). In addition to these mutations, IMS0638, IMS0639 and IMS0643 had three additional SNPs. All mutations in IMS0637-IMS0643 were heterozygous, with the exception of only 3 SNPs. The prevalence of heterozygous SNPs was likely caused by mating of the mutagenized spores of CBS 12357, which resulted in one wild type and one mutated allele at every mutated position. Of the mutations in IMS0637, 34 SNPs and all 5 INDELs affected intergenic regions, 30 SNPs were synonymous, 48 SNPs resulted in amino acid substitutions and 4 SNPs resulted in premature stop codon (data not shown). To the best of our knowledge, none of the 52 non-synonymous SNPs affected genes previously linked to maltotriose utilization. The only copy number variation concerned a duplication of the right subtelomeric region of CHRVIII. Read-mate pairing indicated that the duplicated region was attached to the left arm of CHRII, causing the replacement of left subtelomeric region of CHRII by a non-reciprocal translocation. Although not deemed significant by Pilon, the left subtelomeric region of CHRII indeed showed a lower sequencing coverage. Interestingly, the affected region of CHRII harbored the non-expressed *SeMALT1* gene (Brickwedde et al., 2018. Front Microbiol 9: 1786), although its loss was estimated unlikely to improve maltotriose utilization.

Since the ability to utilize maltotriose in wort emerged only after laboratory evolution, mutations present in IMS0750 and IMS0752 were studied in more detail. IMS0750 and IMS0752 shared 95 SNPs, 3 INDELs and 1 copy number variation with UV-mutants IMS0637-643 (Figure 3A). IMS0750 and IMS0752 were nearly identical: IMS0752 had one silent SNP which was absent in IMS0750. Relative to IMS0637-IMS0643, 16 SNPs and 1 INDEL which were heterozygous became homozygous, and 21 SNPs and 2 INDELs which were heterozygous in IMS0637 were no longer mutated in IMS0750 and 752. In addition, 5 SNPs and 4 copy number variations emerged which were absent in IMS0637-643 (Figure 3A). The 5 SNPs consisted of two heterozygous intergenic SNPs, a heterozygous non-synonymous SNP in the gene of unknown function *SeBSC1*, a homozygous non-synonymous SNP in the putative component of the TOR regulatory pathway *SeMDS3*, and a heterozygous non-synonymous SNP in the vacuolar targeting gene *SePEP1.* Changes in copy number affected several regions harboring *SeMALT* genes: a duplication of 550 bp of CHRII including *SeMALT1* (coordinates 8950 to 9500), a duplication of the left arm of CHRXIII including *SeMALT3* (coordinates 1-10275), loss of the left arm of CHRXVI (coordinates 1-15350), and loss of 5.5 kb of CHRXVI including *SeMALT4* (coordinates 16850-22300). Analysis of read mate pairing indicated that the copy number variation resulted from a complex set of recombinations between chromosomes II, XIII and XVI. The high degree of similarity of the affected *MAL* loci and their localization in the subtelomeric regions made exact reconstruction of the mutations difficult. Therefore, IMS0637 and IMS0750 were sequenced using long-read sequencing on ONT's MinION platform, and a *de novo* genome assembly was made for each strain. Comparison of the resulting assemblies to the chromosome-level assembly of CBS 12357 indicated that two recombinations had occurred. Both in IMS0637 and IMS0750, an additional copy of the last 11500 nucleotides of the right arm of chromosome VIII had replaced the first 11400 nucleotides of one of the two copies of the left arm of chromosome II (Figure 3B). This recombination was consistent with the copy number changes of the affected regions in IMS0637-IMS0643, IMS0750 and IMS0752 and resulted in the loss of one copy of the MAL locus harboring *SeMALT1.* In addition, the genome assembly of IMS0750 indicated the replacement of both copies of the first 22.3 kbp of CHRXVI by complexly rearranged sequences from CHRII, CHRXVIII and CHRXVI. The recombined region consisted precisely of the first 10,273 nucleotides of the left arm of CHRIII, followed by 693 nucleotides from CHRII, 1,468 nucleotides from CHRXVI and 237 nucleotides from CHRXIII (Figure 3B). The recombinations were non reciprocal, as the regions present on the recombined chromosome showed increased sequencing coverage while surrounding regions were unaltered. This recombination resulted in the loss of the canonical *MAL* locus harboring *SeMALT4* on chromosome XVI. However, the recombined sequence contained a chimeric open reading frame consisting of the beginning of *SeMALT4* from CHRXVI, the middle of *SeMALT1* from CHRII and the end of *SeMALT3* from CHRXIII (Figure 3C). To verify this recombination, the ORF was PCR amplified using primers binding on the promotor of *SeMALT4* and the terminator of *SeMALT3.* As expected, a band was obtained for IMS0750, but not for CBS 12357. Sanger sequencing of the amplified fragment confirmed the chimeric organization of the new allele open reading frame, which we named *SeMALT413.* The sequence of *SeMALT413* encoded a full length protein with 100% identity to *SeMALT4* for nucleotides 1-434 and 1113-1145, 100% similarity to *SeMALT1* for nucleotides 430-1122 and 100% similarity to *SeMALT3* for nucleotides 1141-1842 (Figure 3C). Since nucleotides 1123-1140 showed only 72% similarity with *SeMALT1* and 61% similarity with *SeMALT3,* these nucleotides represent an additional introgression from CHRXVI which was not detected previously (Figure 3B). While the first 434 nucleotides could be clearly attributed to *SeMALT4* due to a nucleotide difference with *SeMALT2,* the nucleotides 1123-1140 are identical in *SeMALT2* and *SeMALT4,* therefore the sequence could also have come from *SeMALT2* on CHRV. Notably, *SeMALT413* had a sequence identity of only 85 to 87% with the original *SeMALT* genes, and the corresponding protein sequence exhibited between 52-88% similarity. Therefore, we postulated that the recombined *SeMalt413* transporter might have an altered substrate specificity and might be responsible for the observed maltotriose utilization.

In order to investigate the tertiary structure of the chimeric *SeMALT413* gene, a prediction was made using SWISS-MODEL based on structural-homology with the bacterial xylose proton symporter XylE from *Escherichia coli* (Lam et al., 1980. J Bacteriol 143: 396-402), a reference previously used to model the structure of ScAgt1 (Henderson and Poolman, 2017. Sci Rep 7: 14375). As maltose transporters in *Saccharomyces,* XylE is a proton-symporter with a trans membrane domain composed of 12 α-helixes belonging to the major facilitator superfamily, similarly to *Se*Malt413 (data not shown). The predicted structure of *Se*Malt413 revealed that 1 α-helix was formed exclusively by residues from *Se*Malt4, 4 α-helixes were formed exclusively by residues from *Se*Malt1 and 5 α-helixes were formed exclusively by residues from *Se*Malt3 (Figure 3D). In addition, 2 α-helixes were composed of residues from more than one transporter. Since the first 100 amino acids were excluded from the model due to absence of similar residues in the xylose symporter reference model, the contribution of the *Se*Malt4 sequence was underestimated. The predicted structure of *Se*Malt413 was highly similar to the predicted structures of *Se*Malt1, *Se*Malt3 and *Se*Malt4, indicating it retained the general structure of a functional maltose transporter (data not shown). While marginal structural differences were identified, it remained unclear if these could result in the ability to transport maltotriose, since it is unknown which residues determine the substrate-specificity of such transporters (Henderson and Poolman, 2017. Sci Rep 7: 14375). Furthermore, the ability to utilize maltotriose likely depends on the chemical properties of the residues determining substrate specificity.

### Introduction of the SeMALT413 gene in wildtype CBS 12357 enables maltotriose utilization

To test its functionality and substrate-specificity, *SeMALT413* was overexpressed in the wild type *S. eubayanus* CBS 12357. The putative *SeMALT413* maltotriose transporter was amplified from IMS0750 by PCR (data not shown) and integrated in the plasmid backbone of p426-TEF-amdS between a constitutively expressed *Sc*TEF1 promotor and the *Sc*CYC1 terminator, by "Gibson" assembly (Gibson et al., 2009. Nat Methods 6: 343). The resulting pUD814 plasmid was verified by Sanger sequencing, which confirmed that its *Se*MALT413 ORF was identical to the recombined ORF found in the nanopore assembly of IMS0750 (Figure 3C). The plasmid pUDP052 expressing cas9 and a gRNA targeting *SeSGA1* was previously used successfully for gene integration at the *SeSGA1* locus in CBS 12357 (Brickwedde et al., 2018. Front Microbiol 9: 1786). Therefore, a repair fragment was amplified from pUD814 which contained the *ScTEF1*pr*-SeMALT413-ScCYC1*ter expression cassette flanked by 40 bp homology arms for integration at the *SeSGAl* locus (Figure 4A). The wild type *S. eubayanus* CBS 12357 was transformed with pUDP052 and the repair fragment, resulting in replacement of the *SeSGA1* locus by the *SeMALT413* gene (Figure 4A). As a control, a repair fragment containing the wild type *SeMALT2* ORF between the ScTEF1 promotor and the ScCYC1 terminator was amplified from pUD480 and integrated in a similar manner. The resulting strains IMX1941 (ScTEF1pr-SeMALT2-ScCYC1ter) and IMX1942 (S*cTEF1*pr*-SeMALT413-ScCYC1*ter) were characterized and compared to wild type CBS 12357 and the evolved mutant IMS0750 on SM with different carbon sources. Growth rates were determined based on OD₆₆₀ measurements at regular intervals. On glucose, IMX1941 and IMX1942 grew with a specific growth rate of 0.25 ± 0.01 h⁻¹ while IMS0750 grew faster with a specific growth rate of 0.28 ± 0.01 h⁻¹. Glucose was completely consumed after 33 hours (Figure 4B). On maltose, CBS 12357 and IMX1941 grew with a specific growth rate of 0.19 ± 0.01 h⁻¹, IMX1942 grew with a specific growth rate of 0.18 ± 001 h⁻¹ and IMS0750 grew slower with a specific growth rate of 0.17 ± 0.01 h⁻¹. Maltose was completely consumed after 43 hours (Figure 4C). On maltotriose, only the evolved mutant IMS0750 and reverse engineered strain IMX1942 (*ScTEF1*pr*-SeMALT413-ScCYC1*ter) were able to grow and consume maltotriose. Whereas IMS0750 grew exponentially with a growth rate of 0.19 ± 0.01 h⁻¹ and consumed ± 55% of maltotriose, IMX1942 grew with a growth rate of just 0.03 ± 0.00 h⁻¹ and consumed 45% of the maltotriose after 172 hours, demonstrating functionality of the chimeric *Se*Malt413 transporter (Figure 4D). However the growth on maltotriose after overexpressing *SeMALT413* did not match that of the evolved strain IMS0750. In addition, IMS0750 displayed increased glucose uptake but decreased maltose uptake, suggesting there might be an evolutionary trade-off favoring glucose and maltotriose at the expense of maltose.

### Applicability of a maltotriose-consuming S. eubayanus strain for lager beer brewing

As *S. eubayanus* strains are currently used for industrial lager beer brewing (Brickwedde et al., 2018. Front Microbiol 9: 1786), the evolved strain IMS0750 and its parental strain CBS 12357 were tested in 7-L fermenters on high-gravity 17° Plato wort in duplicate (Figure 5). The reverse engineered strain IMX1942 was not tested, since its genetically modified nature precludes industrial use due to customer acceptance issues (Varzakas et al., 2007. Crit Rev Food Sci Nutr 47: 335-61). After 333 h, IMS0750 had completely consumed all glucose and maltose, and the concentration of maltotriose had dropped from 1.93% m/v) to 0.47% (m/v) (Figure 5). This 75% reduction in maltotriose exceeded the reduction of 60% previously achieved in bottles (from 10.5 g L⁻¹ to 4.3 g L⁻¹, Figure 2D). In contrast, CBS 12357 did not utilize any maltotriose. In addition to the improved maltotriose utilization, IMS0750 expressed improved maltose consumption: all maltose was depleted in less than 200 h, while CBS 12357 had depleted maltose only after 333 h (Figure 5). In accordance with the improved sugar utilization, the final concentration of ethanol was 18.5% higher for IMS0750 than for CBS 12357 (Figure 5). To further explore brewing-related characteristics of IMS0750, the concentration of several aroma-defining esters, higher alcohols and vicinal diketones were monitored. The final concentrations of esters and higher alcohols were predominantly higher in IMS750 culture supernatant, although only the increased concentration of isoamyl acetate was statistically significant (data not shown). In addition, esters and alcohols accumulated faster in IMS0750 than in CBS 12357, likely due to the faster sugar consumption.

Altogether, these results indicate that IMS0750 is able to utilize maltotriose under industrial conditions and suggests that it might express a broader range of improved characteristics for brewing.

## Claims

1. A mutant *Saccharomyces* yeast that is able to ferment maltotriose, preferably a mutant *S. eubayanus* yeast.

2. The mutant *Saccharomyces* yeast of claim 1, comprising a chimeric transporter gene in which part of a first coding gene sequence is translocated adjacent to part of a second coding gene sequence such that the produced chimeric protein harbors part of said first gene product and part of said second gene product.

3. The mutant *S. eubayanus* yeast of claim 1 or claim 2, comprising sequence elements from *SeMALT1, SeMALT2, SeMALT3* and/or *SeMALT4,* preferably *SeMALT4*/*SeMALT1*/*SeMALT2* or *4*/*SeMALT3.*

4. The mutant *S. eubayanus* yeast of any one of claims 1-3, having a chimeric maltose transporter gene comprising nucleotides 1-434 of *SeMALT4,* nucleotides 430-1122 of *SeMALT1,* nucleotides 1113-1145 of *SeMALT2* or *SeMALT4,* and nucleotides 1141-1842 of *SeMALT3,* as depicted in Figure 3C.

5. The mutant *S. eubayanus* yeast of any one of claims 1-4, which has a reduced decarboxylation activity of phenolic acids, preferably is not producing 4-vinyl guaiacol.

6. A method for producing a hybrid yeast, comprising
a) providing the mutant *S. eubayanus* yeast of any one of claims 1-5 as a first parent, and a second yeast as a second parent, which said second parent differs from the first parent,
b) hybridizing cells from the first parent with cells from the second parent
c) identifying a resulting hybrid organism.

7. The method of claim 6, wherein the second parent is a yeast of the *Saccharomyces* sensu stricto complex.

8. The method of claim 6 or claim 7, wherein cells from the first and/or second parent are labeled with a fluorescent dye, prior to hybridizing the cells.

9. The method of any one of claims 6-8, wherein the hybridization is performed at a temperature that is at least 5 °C below the optimal growth temperature of the first and/or the second parent.

10. A hybrid yeast, produced by the method of any one of claims 6-9.

11. A method of producing a fermented beer product, comprising the steps of:
adding a fermentative yeast of any one of claims 1-5 or 10 into a wort, and
at least partially fermenting said wort to produce a fermented beer product.

12. The method of claim 11, wherein the fermentative yeast comprises a mutation resulting in inactivation of at least one of the genes *PAD1* and *FDC1,* and/or inactivation of a gene encoding a protein involved in uptake of a phenolic acid, preferably ferulic acid, or involved in export of a decarboxylated phenolic compound, preferably 4-vinyl guaiacol.

13. The method of claim 11 or 12, wherein the produced fermented beer product is beer, preferably a lager beer.

14. The method of any one of claims 11-13, wherein alcohol content of the fermented beer product is reduced after fermentation, preferably by rectification evaporation.

15. A fermented beer product that is produced by the methods of any one of claims 11-14.

16. Use of the mutant *Saccharomyces eubayanus* yeast of any one of claims 1-5 for producing a hybrid yeast.

17. Use of the mutant *Saccharomyces eubayanus* yeast of any one of claims 1-5, or the hybrid yeast of claim 10, for producing a fermented beer product, preferably a beer, more preferably lager beer.
